# EUROPEAN PATENT SPECIFICATION

(11) **EP 1 953 227 B1**
(45) Date of publication and mention of the grant of the patent: **13.08.2014**
(21) Application number: 06833114.9
(22) Date of filing: 22.11.2006
(51) Int. Cl.: C12N 15/09, A61P 7/02, A61P 29/00, C07K 7/06, A61K 38/00

(54) **LIPOPOLYSACCHARIDE- OR LIPID A-BINDER, AND NOVEL PEPTIDE**
LIPOPOLYSACCHARID- ODER LIPID-A-BINDEMITTEL UND NEUES PEPTID
LIANT DE LIPOPOLYSACCHARIDE OU DE LIPIDE A, ET NOUVEAU PEPTIDE

(30) Priority: 24.11.2005 JP 2005339040; 07.08.2006 WO PCT/JP2006/315613
(43) Date of publication of application: 06.08.2008
(73) Proprietor: Peptide Door Co., Ltd., Takasaki-shi, Gunma 370-0854 (JP)
(72) Inventor: MATSUMOTO, Megumi, Tomioka-shi, Gunma 370-2462 (JP); SUZUKI, Masatsugu, Takasaki-shi, Gunma 370-0857 (JP)
(74) Representative: Cohausz & Florack
(86) International application number: PCT/JP2006/323292
(87) International publication number: WO 2007/060979

(56) References cited:
- WO-A1-2005/047313
- WO-A2-02/28888
- JP-A- 2004 187 563
- JP-A- 2004 189 657
- JP-A- 2004 189 658
- JP-A- 2005 532 130
- DATABASE Geneseq [Online] 23 September 2004 (2004-09-23), "IgG detection-related IgG binding peptide SeqID248." XP002579940 retrieved from EBI accession no. GSP:ADQ08555 Database accession no. ADQ08555
- DATABASE Geneseq [Online] 23 September 2004 (2004-09-23), "IgG purification-related IgG binding peptide SeqID248." XP002579941 retrieved from EBI accession no. GSN:ADQ07295 Database accession no. ADQ07295
- DATABASE Geneseq [Online] 23 September 2004 (2004-09-23), "IgG purification/detection-related IgG binding peptide SeqID248." XP002579942 retrieved from EBI accession no. GSN:ADQ28580 Database accession no. ADQ28580
- ZHU Y. ET AL: 'Sequence and structural diversity in endotoxin-binding dodecapeptides' BIOCHIM. BIOPHYS. ACTA vol. 1611, no. 1-2, 2003, pages 234 - 242, XP003012681
- ONO S. ET AL: 'Jikken Koza 62 Haikesshosei Shock model Sakuseiho' SURG. FRONT vol. 9, no. 2, 2002, pages 157 - 161, XP003012682
- NODA K. ET AL: 'Selection of peptides that bind to the core oligosaccharide of R-form LPS from a phage-displayed heptapeptide library' FEMS MICROBIOLOGICAL LETTERS vol. 205, no. 2, 2001, pages 349 - 354, XP003012683

## Description

### TECHNICAL FIELD

The present invention relates to a lipopolysaccharide and/or lipid A binding agent and a novel peptide.

### BACKGROUND ART

Lipopolysaccharides (LPS) are located in the outer membrane of Gram-negative bacteria. When a patient is infected with Gram-negative bacteria, it is known that LPS released from the bacteria to the body induces sepsis. In this process, LPS is recruited by Toll Like Receptor 4 (TLR4) located on the cell membrane via an LPS binding protein (LBP) in blood plasma, and an inflammatory response mainly based on a nuclear factor κB (NFkB) pathway is induced. Not only fever or inflammation in several portions, but also disseminated intravascular coagulation (DIC) in blood capillaries is induced, to bring death from irreversible reactions such as multiple organ failure.

For sepsis due to Gram-negative bacteria or shock caused by sepsis, a medicament which neutralizes the LPS toxin by binding to LPS, or an LPS removal column for extracorporeal circulation is desired. A potent antibiotic against Gram-negative bacteria, polymyxin B (PMB), was thought to be a neutralizing medicament, but could not be administered to the blood because of its nephrotoxicity and neurotoxicity. Further, clinical trials of two anti-LPS antibodies were conducted in the United States, but failed. As the LPS removal column for extracorporeal circulation, for example, Toraymyxin (Toray) is known, but the manufacturing process must be strictly controlled because of the use of toxic PMB, and thus, the cost is high.

A peptide consisting of the amino acid sequence of SEQ ID NO: 9 described in the present specification is known, for example, as disclosed in patent reference 1. Patent reference 1 discloses that the peptide exhibits a binding activity to an Fc fragment derived from feline IgG.

[patent reference 1] Japanese Unexamined Patent Publication (Kokai) No. 2004-189657

"IgG detection related IgG binding peptide SeqID248 EBI accession no. ADQ08555", "IgG detection related IgG binding peptide SeqID248 EBI accession no. ADQ07295" and "IgG detection related IgG binding peptide SeqID248 EBI accession no. ADQ28580", disclose the same peptide with the amino acid sequence "KNYSSSISSIHA" (SEQ ID NO: 9) as a useful agent for purifying IgGs. WO 02/288888 A2 describes peptides that mimic epitopes of meningococcal lipooligosaccharides as well as the use of these peptides in the treatment and diagnosis of meningococcal diseases. WO 2005/047313 A1 describes methods for lipid A or lipopolysaccharide binding by peptides.

### DISCLOSURE OF INVENTION

### PROBLEMS TO BE SOLVED BY THE INVENTION

The above-mentioned nephrotoxicity and neurotoxicity of PMB was reported by, for example, Danner, R.L. et al. [Purification, toxicity, and antiendotoxin activity of polymyxin B nonapeptide., Antimicrob Agents Chemother. 1989 Sep;33(9):1428-1434 (PMID 2554795)]. The above clinical trials of anti-LPS antibodies in the United States were reported by, for example, Angus, D.C. et al. [E5 murine monoclonal antiendotoxin antibody in gram-negative sepsis: a randomized controlled trial. E5 Study Investigators., JAMA.2000 Apr 5;283(13):1723-1730 (PMID 10755499)] or Derkx, B. et al. [Randomized, placebo-controlled trial of HA-1A, a human monoclonal antibody to endotoxin, in children with meningococcal septic shock. European Pediatric Meningococcal Septic Shock Trial Study Group., Clin Infect Dis. 1999 Apr;28(4):770-777 (PMID 10825037)].

An object of the present invention is to provide a Lipopolysaccharide (LPS) and/or lipid A binding agent which may be used as, for example, an LPS and/or lipid A neutralizing agent or an LPS and/or lipid A removing agent, instead of the known PMB and anti-LPS antibodies.

### MEANS FOR SOLVING THE PROBLEMS

The object may be solved by the present invention, that is, a method of binding lipopolysaccharide and/or lipid A, said method comprising the step of bringing lipopolysaccharide and/or lipid A into contact with a peptide selected from the group consisting of
(1) a peptide exhibiting a lipopolysaccharide and/or lipid A binding activity, and comprising the amino acid sequence of SEQ ID NO: 1, or an amino acid sequence in which one to six amino acids are deleted, substituted, and/or added in the amino acid sequence of SEQ ID NO: 1,
(2) a peptide exhibiting a lipopolysaccharide and/or lipid A binding activity, and comprising the amino acid sequence of SEQ ID NO: 2, or an amino acid sequence in which one amino acid is deleted, substituted, and/or added in the amino acid sequence of SEQ ID NO: 2,
(3) a peptide exhibiting a lipopolysaccharide and/or lipid A binding activity, and comprising the amino acid sequence of SEQ ID NO: 4, or an amino acid sequence in which one to six amino acids are deleted, substituted, and/or added in the amino acid sequence of SEQ ID NO: 4, and
(4) a peptide exhibiting a lipopolysaccharide and/or lipid A binding activity, and comprising the amino acid sequence of SEQ ID NO: 5,
or a derivative of the peptide,
wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a non-naturally occurring amino acid having properties similar to the original amino acid, or a combination, thereof, and wherein the derivative exhibits the lipopolysaccharide and/or lipid A binding activity
provided that the method is not a method for treatment of the human or animal body by surgery or therapy or a diagnostic method practised on the human or animal body.

The present invention relates any one of the peptides (1) to (4) or derivative thereof, polynucleotide encoding the peptide of claim 1, or an expression vector comprising the polynucleotide, for use as a medicament.

The present invention relates to any one of the peptides (1) to (4) or derivative thereof, polynucleotide encoding any one of the peptides (1) to (4), or an expression vector comprising the polynucleotide, for treating sepsis.

The present invention relates to a compound for use as a medicament, wherein the compound is any one of the peptides (1) to (4) or derivative thereof, a polynucleotide encoding any one of the peptides (1) to (4), or an expression vector comprising the polynucleotide and which includes therapeutically neutralizing lipopolysaccharide and/or lipid A.

The present invention relates to a compound for use in treating sepsis, wherein the compound is any one of the peptides (1) to (4) or derivative thereof, a polynucleotide encoding any one of the peptides (1) to (4), or an expression vector comprising the polynucleotide.

The present invention relates to a method of analyzing lipopolysaccharide and/or lipid A, characterized by using any one of the peptides (1) to (4) or derivative thereof.

The present invention relates to a method of analyzing Gram-negative bacteria, characterized by using any one of the peptides (1) to (4) or derivative thereof.

The present invention relates to a peptide selected from the group consisting of
(1) a peptide exhibiting a lipopolysaccharide and/or lipid A binding activity, and comprising the amino acid sequence of SEQ ID NO: 1, or an amino acid sequence in which one to six amino acids are deleted, substituted, and/or added in the amino acid sequence of SEQ ID NO: 1,
(2) a peptide exhibiting a lipopolysaccharide and/or lipid A binding activity, and comprising the amino acid sequence of SEQ ID NO: 2, or an amino acid sequence in which one amino acid is deleted, substituted, and/or added in the amino acid sequence of SEQ ID NO: 2,
(3) a peptide exhibiting a lipopolysaccharide and/or lipid A binding activity, and comprising the amino acid sequence of SEQ ID NO: 4, or an amino acid sequence in which one to six amino acids are deleted, substituted, and/or added in the amino acid sequence of SEQ ID NO: 4, and
(4) a peptide exhibiting a lipopolysaccharide and/or lipid A binding activity, and comprising the amino acid sequence of SEQ ID NO: 5,
or a derivative thereof, with the proviso that the peptide is not a peptide consisting of the amino acid sequence of SEQ ID NO: 9, wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a nonnaturally occurring amino acid having properties similar to the original amino acid, or a combination, thereof, and wherein the derivative exhibits the lipopolysaccharide and/or lipid A binding activity.

The present invention relates to a polynucleotide encoding any one of the peptides (1) to (4), with the proviso that the peptide is not a peptide consisting of the amino acid sequence of SEQ ID NO: 9.

The present invention relates to a n expression vector comprising the polynucleotide encoding any one of the peptides (1) to (4).

The present invention relates to a pharmaceutical composition comprising any one of the peptides (1) to (4), a derivative thereof, a polynucleotide encoding any one of the peptides (1) to (4), or an expression vector comprising the polynucleotide, and a pharmaceutically or veterinarily acceptable carrier or diluent, with the proviso that the peptide is not a peptide consisting of the amino acid sequence of SEQ ID NO: 9 wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a non-naturally occurring amino acid having properties similar to the original amino acid, or a combination, thereof, and wherein the derivative exhibits the lipopolysaccharide and/or lipid A binding activity.

### EFFECTS OF THE INVENTION

According to the present invention, a novel lipopolysaccharide and/or lipid A binding agent can be provided. The lipopolysaccharide and/or lipid A binding agent of the present invention can be used as, for example, a lipopolysaccharide and/or lipid A removing agent or a lipopolysaccharide and/or lipid A neutralizing agent.

### BEST MODE FOR CARRYING OUT THE INVENTION

Hereinafter, the above peptides (1) to (4) and the derivatives thereof are collectively referred to as the LPS and/or lipid A binding peptide. The term, peptide, as used herein includes an oligopeptide and a polypeptide.

The term, LPS and/or lipid A binding activity, as used herein means an activity of specifically binding to at least one of LPS or lipid A, preferably an activity of specifically binding to both LPS and lipid A. LPS is a compound composed of a polysaccharide portion and a phospholipid portion (i.e., lipid A). While the polysaccharide portion shows diversity among species or stains, the lipid A portion has a similar structure. The lipid A portion plays a critical role in an LPS toxicity.

Whether or not a certain peptide exhibits the LPS and/or lipid A binding activity can be easily judged by a conventional method. As the conventional method, there may be mentioned, for example, a method of analyzing a binding of the peptide to LPS or lipid A (preferably lipid A alone, or a combination of LPS and lipid A) immobilized on an appropriate carrier, such as an ELISA plate or a bead carrier (see Example 1 or Example 3), or a method based on surface plasmon resonance (SPR) [for example, a method using a BIACORE system such as BIACORE 2000 (BIACORE)].

A number of amino acid residues contained in the LPS and/or lipid A binding peptide is not limited, so long as the peptide exhibits the LPS and/or lipid A binding activity. The LPS and/or lipid A binding peptide which may be used in the present invention may be composed of, for example, 5 to 100 amino acid residues, preferably 5 to 61 amino acid residues, more preferably 5 to 37 amino acid residues, still more preferably 5 to 25 amino acid residues, most preferably 5 to 13 amino acid residues. The peptide may be composed of an amino acid sequence which shows the LPS and/or lipid A binding activity per se (i.e., a fundamental unit sequence), or a repetitive sequence consisting of fundamental unit sequences). The repetitive sequence may be composed of a type of fundamental unit sequence alone, or may be a combination of two or more types of fundamental unit sequences. A binding force of the peptide may be increased by repeating fundamental unit sequences. When a peptide having a weak binding force is used, the total binding force may be increased by immobilizing plural peptides on a carrier with highly populated and adjacent molecules (such as polylysine) having a side chain showing a high reactivity. This plural peptide immobilized carrier may be prepared in accordance with, for example, a method for preparing a column for IgG purification [Fassina G et al, Protein A mimetic peptide ligand for affinity purification of antibodies. J. Mol. Recognit 1996, 9(5-6), 564-569].

As the above LPS and/or lipid A binding peptide (1), that is, a peptide exhibiting a lipopolysaccharide and/or lipid A binding activity, and comprising the amino acid sequence of SEQ ID NO: 1, or an amino acid sequence in which one or several amino acids are deleted, substituted, and/or added in the amino acid sequence of SEQ ID NO: 1, there may be mentioned, for example,
a peptide consisting of the amino acid sequence of SEQ ID NO: 1;
a peptide exhibiting the LPS and/or lipid A binding activity, and consisting of an amino acid sequence in which one or more appropriate amino acid sequences are added to the N-terminus and/or the C-terminus of the amino acid sequence of SEQ ID NO: 1; or
a peptide exhibiting the LPS and/or lipid A binding activity, and comprising an amino acid sequence in which one or several amino acids are deleted, substituted, and/or added in the amino acid sequence of SEQ ID NO: 1.

To maintain the functions of the original peptide, the amino acid to be substituted is preferably an amino acid having properties similar to those of the original amino acid. For example, amino acids belonging to each of the following groups have properties similar to those of other members in the group. When these amino acids are substituted with other amino acids in the same group, the essential functions of the original protein are often maintained. Such amino acid substitution is called a conservative substitution, and is known as a method for changing an amino acid sequence while maintaining the polypeptide functions. Nonpolar amino acids: Ala, Val, Leu, Ile, Pro, Met, Phe, and Trp Uncharged amino acids: Gly, Ser, Thr, Cys, Tyr, Asn, and Gln Acidic amino acids: Asp and Glu Basic amino acids: Lys, Arg, and His

The amino acid sequence of SEQ ID NO: 1 includes the following amino acid sequences in which the amino acids X at the first and 11th positions in the amino acid sequence of SEQ ID NO: 1 are independently basic amino acids K, R, or H:
KNYSSSISSIKA (SEQ ID NO: 7)
KNYSSSISSIRA (SEQ ID NO: 8)
KNYSSSISSIHA (SEQ ID NO: 9)
RNYSSSISSIKA (SEQ ID NO: 10)
RNYSSSISSIRA (SEQ ID NO: 11)
RNYSSSISSIHA (SEQ ID NO: 12)
HNYSSSISSIKA (SEQ ID NO: 13)
HNYSSSISSIRA (SEQ ID NO: 14)
HNYSSSISSIHA (SEQ ID NO: 15)

A peptide consisting of the amino acid sequence of SEQ ID NO: 9 is a known peptide disclosed in, for example, Japanese Unexamined Patent Publication (Kokai) No. 2004-189657, and exhibits the LPS and/or lipid A binding activity, as shown in Examples described below. Japanese Unexamined Patent Publication (Kokai) No. 2004-189657 discloses that the peptide exhibits a binding activity to an Fc fragment of feline IgG, but does not disclose the LPS and/or lipid A binding activity.

In the above peptide exhibiting the LPS and/or lipid A binding activity, and consisting of an amino acid sequence in which one or more appropriate amino acid sequences are added to the N-terminus and/or the C-terminus of the amino acid sequence of SEQ ID NO: 1, the amino acid sequence to be added to the N-terminus and/or the C-terminus may be, for example, a linker sequence, a marker sequence, a polypeptide sequence, or other LPS and/or lipid A binding peptide sequences.

The linker sequence may be, for example, a sequence for immobilizing a peptide on a carrier. As the sequence, there may be mentioned, for example, a linker sequence consisting of an amino acid having a thiol group [for example, cysteine (L-cysteine or D-cysteine) or homocysteine], a linker sequence consisting of an amino acid having, as a side chain, a functional group not reacting with an amino group (such as a maleimide group), or a linker sequence in which at least one terminal amino acid is an amino acid having a thiol group or an amino acid having a functional group not reacting with an amino group.

As the marker sequence, a sequence for easily carrying out a confirmation of peptide expression, a confirmation of intracellular localization thereof, a purification thereof, or the like may be used. As the sequence, there may be mentioned, for example, a FLAG tag, a hexa-histidine tag, a hemagglutinin tag, a myc epitope, or a peptide consisting of the amino acid sequence
GGLLLLLLL (SEQ ID NO: 125).
The C-terminal carboxyl group of the peptide of SEQ ID NO: 125 may be amidated, or may be used without the amidation.

As the polypeptide sequence, there may be mentioned, for example, a polypeptide for purification [such as the full-length or a part of glutathione-S-transferase (GST)], a polypeptide for detection [such as the full-length or a part of hemagglutinin or β-galactosidase α peptide (lacZ α)], or a polypeptide for expression (such as a signal sequence).

As the peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 1, there may be mentioned, for example, a peptide consisting of the amino acid sequence
XNYSSSISSIXAC (SEQ ID NO: 16).
In the amino acid sequence of SEQ ID NO: 16, the amino acid X at the first position is a basic amino acid K, R, or H, preferably K, and the amino acid X at the 11th position is a basic amino acid K, R, or H, preferably R or H, more preferably R.

As the above peptide exhibiting the LPS and/or lipid A binding activity, and comprising an amino acid sequence in which one or several amino acids (preferably 1 to 10 amino acids, more preferably 1 to 8 amino acids, still more preferably 1 to 6 amino acids, still more preferably 1 to 4 amino acids, still more preferably 1 to 3 amino acids, still more preferably 1 or 2 amino acids, most preferably 1 amino acid) are deleted, substituted, and/or added in the amino acid sequence of SEQ ID NO: 1, there may be mentioned, for example, the above LPS and/or lipid A binding peptides (2) to (6), as explained in detail below.

As the LPS and/or lipid A binding peptide (2), that is, a peptide exhibiting a lipopolysaccharide and/or lipid A binding activity, and comprising the amino acid sequence of SEQ ID NO: 2, or an amino acid sequence in which one or several amino acids are deleted, substituted, and/or added in the amino acid sequence of SEQ ID NO: 2, there may be mentioned, for example,
a peptide consisting of the amino acid sequence of SEQ ID NO: 2;
a peptide exhibiting the LPS and/or lipid A binding activity, and consisting of an amino acid sequence in which one or more appropriate amino acid sequences are added to the N-terminus and/or the C-terminus of the amino acid sequence of SEQ ID NO: 2; or
a peptide exhibiting the LPS and/or lipid A binding activity, and comprising an amino acid sequence in which one or several amino acids are deleted, substituted, and/or added in the amino acid sequence of SEQ ID NO: 2.

The amino acid sequence of SEQ ID NO: 2 is that consisting of amino acids 1 to 6 in the amino acid sequence of SEQ ID NO: 1. The amino acid sequence of SEQ ID NO: 2 includes the following amino acid sequences in which the amino acid X at the first position in the amino acid sequence of SEQ ID NO: 2 is a basic amino acid K, R, or H:
KNYSSS (SEQ ID NO: 17)
RNYSSS (SEQ ID NO: 18)
HNYSSS (SEQ ID NO: 19)

In the above peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 2, the amino acid sequence to be added to the N-terminus and/or the C-terminus may be, for example, a linker sequence, a marker sequence, a polypeptide sequence, or other LPS and/or lipid A binding peptide sequences, as previously described with respect to the LPS and/or lipid A binding peptide (1).

As the peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 2, there may be mentioned, for example, a peptide consisting of an amino acid sequence selected from the following amino acid sequences:
XNYSSSI (SEQ ID NO: 20)
XNYSSSIS (SEQ ID NO: 21)
XNYSSSISS (SEQ ID NO: 22)
XNYSSSISSI (SEQ ID NO: 23)
XNYSSSISSIX (SEQ ID NO: 24)
XNYSSSISSIXA (SEQ ID NO: 1)
or a peptide consisting of an amino acid sequence selected from the following amino acid sequences in which C is further added to the C-terminus of these amino acid sequences (or SEQ ID NO: 2):
XNYSSSC (SEQ ID NO: 25)
XNYSSSIC (SEQ ID NO: 26)
XNYSSSISC (SEQ ID NO: 27)
XNYSSSISSC (SEQ ID NO: 28)
XNYSSSISSIC (SEQ ID NO: 29)
XNYSSSISSIXC (SEQ ID NO: 30)
XNYSSSISSIXAC (SEQ ID NO: 16)
The amino acid X at the first position in these amino acid sequences is a basic amino acid K, R, or H, preferably K, and the amino acid X at the 11th position is a basic amino acid K, R, or H, preferably R or H, more preferably R.

The above peptide exhibiting the LPS and/or lipid A binding activity, and comprising an amino acid sequence in which one or several amino acids (preferably 1 to 10 amino acids, more preferably 1 to 8 amino acids, still more preferably 1 to 6 amino acids, still more preferably 1 to 4 amino acids, still more preferably 1 to 3 amino acids, still more preferably 1 or 2 amino acids, most preferably 1 amino acid) are deleted, substituted, and/or added in the amino acid sequence of SEQ ID NO: 2 may be, for example, a peptide exhibiting the LPS and/or lipid A binding activity and comprising the amino acid sequence:
NYSSS (SEQ ID NO: 31).
As the peptide, there may be mentioned, for example, a peptide consisting of an amino acid sequence selected from the following amino acid sequences:
NYSSS (SEQ ID NO: 31)
NYSSSI (SEQ ID NO: 32)
NYSSSIS (SEQ ID NO: 33)
NYSSSISS (SEQ ID NO: 34)
NYSSSISSI (SEQ ID NO: 35)
NYSSSISSIX (SEQ ID NO: 36)
NYSSSISSIXA (SEQ ID NO: 37)
   or a peptide consisting of an amino acid sequence selected from the following amino acid sequences in which C is further added to the C-terminus of these amino acid sequences:
NYSSSC (SEQ ID NO: 38)
NYSSSIC (SEQ ID NO: 39)
NYSSSISC (SEQ ID NO: 40)
NYSSSISSC (SEQ ID NO: 41)
NYSSSISSIC (SEQ ID NO: 42)
NYSSSISSIXC (SEQ ID NO: 43)
NYSSSISSIXAC (SEQ ID NO: 44)
The amino acid X at the 10th position in these amino acid sequences is a basic amino acid K, R, or H, preferably R or H, more preferably R.

As the above LPS and/or lipid A binding peptide (3), that is, a peptide exhibiting a lipopolysaccharide and/or lipid A binding activity, and comprising the amino acid sequence of SEQ ID NO: 4, or an amino acid sequence in which one or several amino acids are deleted, substituted, and/or added in the amino acid sequence of SEQ ID NO: 4, there may be mentioned, for example,
a peptide consisting of the amino acid sequence of SEQ ID NO: 4;
a peptide exhibiting the LPS and/or lipid A binding activity, and consisting of an amino acid sequence in which one or more appropriate amino acid sequences are added to the N-terminus and/or the C-terminus of the amino acid sequence of SEQ ID NO: 4; or
a peptide exhibiting the LPS and/or lipid A binding activity, and comprising an amino acid sequence in which one or several amino acids are deleted, substituted, and/or added in the amino acid sequence of SEQ ID NO: 4.

The amino acid sequence of SEQ ID NO: 4 is a sequence in which the N at the second position is deleted in the amino acid sequence of SEQ ID NO: 1. The amino acid sequence of SEQ ID NO: 4 includes the following amino acid sequences in which the amino acids X at the first and 10th positions in the amino acid sequence of SEQ ID NO: 4 are independently basic amino acids K, R, or H:
KYSSSISSIKA (SEQ ID NO: 58)
KYSSSISSIRA (SEQ ID NO: 59)
KYSSSISSIHA (SEQ ID NO: 60)
RYSSSISSIKA (SEQ ID NO: 61)
RYSSSISSIRA (SEQ ID NO: 62)
RYSSSISSIHA (SEQ ID NO: 63)
HYSSSISSIKA (SEQ ID NO: 64)
HYSSSISSIRA (SEQ ID NO: 65)
HYSSSISSIHA (SEQ ID NO: 66)

As the peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 4, there may be mentioned, for example, a peptide consisting of the following amino acid sequence:
XYSSSISSIXAC (SEQ ID NO: 67)
The amino acid X at the first position in the amino acid sequence of SEQ ID NO: 67 is a basic amino acid K, R, or H, preferably K, and the amino acid X at the 10th position is a basic amino acid K, R, or H, preferably R or H, more preferably R.

As the above peptide exhibiting the LPS and/or lipid A binding activity, and comprising an amino acid sequence in which one or several amino acids (preferably 1 to 10 amino acids, more preferably 1 to 8 amino acids, still more preferably 1 to 6 amino acids, still more preferably 1 to 4 amino acids, still more preferably 1 to 3 amino acids, still more preferably 1 or 2 amino acids, most preferably 1 amino acid) are deleted, substituted, and/or added in the amino acid sequence of SEQ ID NO: 4, there may be mentioned, for example, the above LPS and/or lipid A binding peptides (1) (2), and (4), particularly the LPS and/or lipid A binding peptides (4), as explained in detail below.

As the LPS and/or lipid A binding peptide (4), that is, a peptide exhibiting a lipopolysaccharide and/or lipid A binding activity, and comprising the amino acid sequence of SEQ ID NO: 5, or an amino acid sequence in which one or several amino acids are deleted, substituted, and/or added in the amino acid sequence of SEQ ID NO: 5, there may be mentioned, for example,
a peptide consisting of the amino acid sequence of SEQ ID NO: 5;
a peptide exhibiting the LPS and/or lipid A binding activity, and consisting of an amino acid sequence in which one or more appropriate amino acid sequences are added to the N-terminus and/or the C-terminus of the amino acid sequence of SEQ ID NO: 5; or
a peptide exhibiting the LPS and/or lipid A binding activity, and comprising an amino acid sequence in which one or several amino acids are deleted, substituted, and/or added in the amino acid sequence of SEQ ID NO: 5.

The amino acid sequence of SEQ ID NO: 5 is a sequence consisting of amino acids 1 to 5 in the amino acid sequence of SEQ ID NO: 4. The amino acid sequence of SEQ ID NO: 5 includes the following amino acid sequences in which the amino acid X at the first position in the amino acid sequence of SEQ ID NO: 5 is a basic amino acid K, R, or H:
KYSSS (SEQ ID NO: 68)
RYSSS (SEQ ID NO: 69)
HYSSS (SEQ ID NO: 70)

In the above peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 5, the amino acid sequence to be added to the N-terminus and/or the C-terminus may be, for example, a linker sequence, a marker sequence, a polypeptide sequence, or other LPS and/or lipid A binding peptide sequences, as previously described with respect to the LPS and/or lipid A binding peptide (1).

As the peptide exhibiting the LPS and/or lipid A binding activity, and consisting of an amino acid sequence in which one or more appropriate amino acid sequences are added to the N-terminus and/or the C-terminus (preferably the C-terminus) of the amino acid sequence of SEQ ID NO: 5, there may be mentioned, for example, a peptide consisting of an amino acid sequence selected from the following amino acid sequences:
XYSSSI (SEQ ID NO: 71)
XYSSSIS (SEQ ID NO: 72)
XYSSSISS (SEQ ID NO: 73)
XYSSSISSI (SEQ ID NO: 74)
XYSSSISSIX (SEQ ID NO: 75)
XYSSSISSIXA (SEQ ID NO: 4)
or a peptide consisting of an amino acid sequence selected from the following amino acid sequences in which C is further added to the C-terminus of these amino acid sequences (or SEQ ID NO: 5):
XYSSSC (SEQ ID NO: 76)
XYSSSIC (SEQ ID NO: 77)
XYSSSISC (SEQ ID NO: 78)
XYSSSISSC (SEQ ID NO: 79)
XYSSSISSIC (SEQ ID NO: 80)
XYSSSISSIXC (SEQ ID NO: 81)
XYSSSISSIXAC (SEQ ID NO: 82)
The amino acid X at the first position in these amino acid sequences is a basic amino acid K, R, or H, preferably K, and the amino acid X at the 10th position is a basic amino acid K, R, or H, preferably R or H, more preferably R.

The above peptide exhibiting the LPS and/or lipid A binding activity, and comprising an amino acid sequence in which one or several amino acids (preferably 1 to 10 amino acids, more preferably 1 to 8 amino acids, still more preferably 1 to 6 amino acids, still more preferably 1 to 4 amino acids, still more preferably 1 to 3 amino acids, still more preferably 1 or 2 amino acids, most preferably 1 amino acid) are deleted, substituted, and/or added in the amino acid sequence of SEQ ID NO: 5 may be, for example, a peptide exhibiting the LPS and/or lipid A binding activity and comprising the amino acid sequence:
YSSS (SEQ ID NO: 83).
As the peptide, there may be mentioned, for example, a peptide consisting of an amino acid sequence selected from the following amino acid sequences:
YSSS (SEQ ID NO: 83)
YSSSI (SEQ ID NO: 84)
YSSSIS (SEQ ID NO: 85)
YSSSISS (SEQ ID NO: 86)
YSSSISSI (SEQ ID NO: 87)
YSSSISSIX (SEQ ID NO: 88)
YSSSISSIXA (SEQ ID NO: 89)
or a peptide consisting of an amino acid sequence selected from the following amino acid sequences in which C is further added to the C-terminus of these amino acid sequences:
YSSSC (SEQ ID NO: 90)
YSSSIC (SEQ ID NO: 91)
YSSSISC (SEQ ID NO: 92)
YSSSISSC (SEQ ID NO: 93)
YSSSISSIC (SEQ ID NO: 94)
YSSSISSIXC (SEQ ID NO: 95)
YSSSISSIXAC (SEQ ID NO: 96)
The amino acid X at the 9th position in these amino acid sequences is a basic amino acid K, R, or H, preferably R or H, more preferably R.

The derivatives of the peptides (1) to (4), which may be used as an active ingredient in the present invention, are not limited, so long as they exhibit the LPS and/or lipid A activity. As the derivatives, there may be mentioned, for example, a peptide derivative which may be obtained by modifying an original peptide to develop a stability thereof. As the modification, there may be mentioned, for example, a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a nonnaturally occurring amino acid having properties similar to the original amino acid, or a combination thereof.

As a derivative obtained by the substitution of a D-form amino acid for the N-terminal L-form amino acid, a derivative obtained by a substitution of a D-form basic amino acid is preferable, and a derivative obtained by a substitution of D-Lysine or D-arginine is more preferable.

The LPS and/or lipid A binding agent of the present invention contains the LPS and/or lipid A binding peptide as an active ingredient. Since the LPS and/or lipid A binding peptide exhibits a binding activity to LPS, the LPS and/or lipid A binding agent of the present invention may be used as, for example, an LPS and/or lipid A removing agent (such as an LPS removal column for extracorporeal circulation) or an LPS and/or lipid A neutralizing agent (such as a therapeutic agent for sepsis). Further, the LPS and/or lipid A binding agent of the present invention may be used to analyze LPS and/or lipid A, such as an ELISA method, an imaging of single fluorescent molecules, or an SPR (surface plasmon resonance) method. Since the LPS and/or lipid A binding agent of the present invention binds to LPS located in the outer membrane of Gram-negative bacteria, it may be used in staining, labeling, or analyzing (particularly detecting) of Gram-negative bacteria such as Escherichia coli. The LPS and/or lipid A binding agent of the present invention may contain the LPS and/or lipid A binding peptide as an active ingredient, in any form according to the use thereof.

When the LPS and/or lipid A binding agent of the present invention is used as the LPS and/or lipid A removing agent, as a subject in need of an LPS removal, there may be mentioned, for example, blood plasma, a serum, blood, a dialysis fluid, an infusion, an injection, or various buffers. In this case, the LPS and/or lipid A binding peptide as an active ingredient may be used alone, or preferably as the peptide immobilized on an appropriate carrier. As the carrier, there may be mentioned, for example, silica beads, agarose beads, cellulose beads, magnetic beads, or glass fibers. The LPS and/or lipid A binding peptide may be immobilized on the carrier, for example, by a binding via a disulfide bond or a maleimide method using a maleimide group of the carrier.

When the LPS and/or lipid A binding agent of the present invention is used as the LPS and/or lipid A neutralizing agent, the peptide alone, or together with a pharmaceutically or veterinarily acceptable ordinary carrier or diluent if desired, may be administered to an animal, preferably a mammal, particularly a human. In this case, a polynucleotide encoding the peptide, preferably an expression vector containing the polynucleotide, may be used instead of the peptide [for example, Gene Ther., Development of safe and efficient novel nonviral gene transfer using ultrasound: enhancement of transfection efficiency of naked plasmid DNA in skeletal muscle., 2002 Mar;9(6):372-80]. The neutralizing activity of the LPS and/or lipid A binding peptide may be confirmed, for example, in accordance with a method disclosed in "7. New strategy for endotoxin studies by phage display method, SUZUKI, Masatsugu et al., Endotoxin Studies 7, Igaku Tosho Shuppan Co., Ltd., 2004, p.65-72". In this method, LPS is mixed with the LPS and/or lipid A binding peptide at various concentrations and ratios, and analyzed by a limulus test, a conventional method for measuring a concentration of LPS. The neutralizing activity of the peptide may be evaluated in vitro by comparing the amounts of LPS added with the measuring values and determining the differences therebetween.

When the LPS and/or lipid A binding agent of the present invention is used to analyze LPS and/or lipid A, the analysis can be carried out, for example, by bringing a sample suspected of containing LPS and/or lipid A into contact with the LPS and/or lipid A binding peptide (preferably the peptide immobilized on an appropriate carrier), and analyzing LPS and/or lipid A bound to the peptide. The term, analysis, as used herein includes a detection to judge a presence or absence of a substance to be analyzed, and a measurement to quantitatively or semi-quantitatively determine an amount or activity of a substance to be analyzed.

As the carrier, there may be mentioned, for example, beads (such as silica beads, agarose beads, cellulose beads, or magnetic beads), or a plate (such as an ELISA plate). The LPS and/or lipid A bound to the peptide can be analyzed by, for example, a commercially available kit for measuring endotoxin (such as Endospecy ES-50M set; Seikagaku Corporation), an immunological analysis using an antibody specific to LPS and/or lipid A, or an SPR (surface plasmon resonance) method.

When the LPS and/or lipid A binding agent of the present invention is used to stain or label Gram-negative bacteria therewith, for example, cysteine may be added to the C-terminus of the LPS and/or lipid A binding peptide, and the peptide may be conjugated to, for example, a labeling compound (such as a dye, a fluorescent compound, or a luminescent compound) or a protein (for example, an enzyme such as peroxides, or an Fc portion of an antibody) via the thiol group of the cysteine. More particularly, a fluorescent compound, fluorescein-5-maleimide (PIERCE), may be conjugated to the LPS and/or lipid A binding peptide, and the resulting conjugate may be purified by high performance liquid chromatography or the like to obtain a peptide conjugate for fluorescence staining or labeling. A useful protein, such as an enzyme or an Fc portion of an antibody, may be easily conjugated to the LPS and/or lipid A binding peptide, by using sulfa-SMCC (PIERCE) or the like. The LPS and/or lipid A binding peptide labeled as described above may be mixed with Gram-negative bacteria to stain or label the bacteria.

When the LPS and/or lipid A binding agent of the present invention is used to analyze Gram-negative bacteria, the analysis (particularly detection) may be carried out, for example, by bringing a sample suspected of containing Gram-negative bacteria into contact with the LPS and/or lipid A binding peptide (preferably the peptide immobilized on an appropriate carrier), and analyzing Gram-negative bacteria bound to the peptide. The Gram-negative bacteria bound to the peptide can be analyzed by, for example, an immunological analysis using an antibody specific to the Gram-negative bacteria, a detection of fluorescence derived from a fluorescent dye conjugated to the peptide, or a detection based on an enzyme-substrate reaction of horseradish peroxidase (HRP) or alkaline phosphatase conjugated to the peptide.

### Examples

The present invention now will be further illustrated by, but is by no means limited to, the following Examples.

### Example 1: Screening for LPS and/or lipid A binding peptides by phage display method

In this example, a phage display method was used to carry out a screening for peptides capable of binding to both lipid A prepared from E. coli (Lipid A; E.coli K12, D31m4 <Primarily diphosporyl>; Funakoshi) and LPS prepared from E. coli (E.coli K12 D31 m4 (Re); Funakoshi).

As libraries used in the phage display method, two libraries in which peptides were randomly displayed at the N-terminus of a minor protein pIII located on the surface of an M13 phage were prepared in accordance with Smith, G. P., Science, 288, 1315-1317 (1985), J. K. Scott and G. P. Smith, Science, 249, 386-390 (1990), and United States Patent No. 5,223,409 (Ladner et al.). In these libraries, peptides consisting of seven or twelve random amino acids were displayed. The concrete procedures were carried out in accordance with Japanese Unexamined Patent Publication (Kokai) No. 2004-189657.

The binding activities of the phage libraries to the targets (i.e., lipid A and LPS) were evaluated by an enzyme-linked immunosorbent assay (ELISA). More particularly, each target was immobilized on a 96-well plate [a 96-well PolySorp plate (NUNC) for lipid A and a 96-well MediSorp plate (NUNC) for LPS] by dissolving lipid A or LPS (100 µg/mL) in a phosphate-buffered saline (pH 7.4; hereinafter referred to as PBS), dispensing 50 µL of the solution to the plate, and incubating the plate at 4°C overnight. A peroxidase-labeled anti-M13 antibody (anti M13 antibody HRP monoclonal conjugate; Amersham Biosceiences) was used as a labeled antibody, and 2,2'-azino-bis(3-ethylbenzothiazoline-6-sulfonic acid) diammonium salt (ABTS; Wako Pure Chemicals) was used as an enzyme substrate.

A resulting phage which displayed a peptide capable of binding to both lipid A and LPS was sequenced to obtain the amino acid sequence:
KNYSSSISSIHA (SEQ ID NO: 9).

### Example 2: Confirmation of binding activity to LPS and lipid A

In this example, a BIACORE system (BIACORE 2000; BIACORE) was used to confirm the binding activity of the peptide (hereinafter referred to as Li5) comprising the amino acid sequence of SEQ ID NO: 9 determined in Example 1 to LPS and lipid A. In this connection, any buffers, distilled water, equipment, and the like used in the following procedures were endotoxin-free, unless otherwise specified.

To introduce a thiol group at the C-terminus of the amino acid sequence of SEQ ID NO: 9, a peptide (hereinafter referred to as Li5 C) consisting of the amino acid sequence:
KNYSSSISSIHAC (SEQ ID NO: 110),
in which cysteine was added, was synthesized.

The synthesized peptide Li5 C was immobilized on a flow cell (hereinafter referred to as Fc), Fc2, of a sensor chip (BIACORE Sensor Chip CM5 ; BIACORE) by thiol coupling. As a control, cysteine was immobilized on Fc1. For comparison, polymyxin B (PMB) was immobilized on Fc4 via an amino group by amine coupling. As a control for PMB, ethanolamine was immobilized on Fc3.

Various concentrations of lipid A solutions and LPS solutions [concentration = 100 µg/mL, 50 µg/mL, 25 µg/mL, and 12.5 µg/mL; Buffer HBS (0.01 mol/L HEPES, pH7.4, 0.15 mol/L NaCl, and 3 mmol/L EDTA)] as an analyte were applied to the sensor chip in order of an increasing concentration to carry out the measurement. As a result, the average of a dissociation constant (KD) of the peptide Li5 C to lipid A or LPS was 10⁻⁷ to 10⁻⁹, and it was confirmed that the peptide Li5 C strongly binds to lipid A and LPS. In addition, the binding activity (KD) of PMB for comparison was 10⁻⁷ to 10⁻¹⁰, and it was found that the binding activity of the peptide Li5 C is similar to that of PMB, a known antibiotic. In this connection, it was considered that the wide ranges of the measurement values were due to the micelle state of LPS or lipid A.

### Example 3: Evaluation of LPS and/or lipid A binding peptide-immobilized beads in LPS removal

### (3-1) Immobilization of peptide Li5 C on bead carrier

In this example, a reaction was carried out by adding a solvent to silica gel with acid chloride (Propionyl chloride functionalized silica gel 200-400 mesh; Sigma-Aldrich)(hereinafter referred to as bead carrier) to immobilize the peptide Li5 C on the bead carrier via the thiol group of the C-terminal cysteine of the peptide Li5 C. In this connection, any buffers, distilled water, equipment, and the like used in the following procedures were endotoxin-free, unless otherwise specified.

More particularly, the bead carrier (0.5 g) was weighed out and put into a dry heat sterilized test tube. To the test tube, 2-iodoethanol (Wako Pure Chemicals; 1 mL) was added to react the hydroxyl group of iodoethanol with acid chloride conjugated to the bead carrier, to form a covalent bond. In this reaction, hydrogen chloride (HCl) would be produced as a by-product. Pyridine (Wako Pure Chemicals; a compound containing amine base; 100 µL) was added to this reaction system to avoid the side reaction of iodoethanol. The test tube was covered with parafilm and the whole was mixed well. The reaction was carried out at room temperature for 4 hours while gently stirring.

The reaction products were transferred to an Econo-Column (BIO-RAD), well washed with distilled water, and equilibrated with a coupling buffer (50 mmol/L Tris-HCl, 5 mmol/L EDTA·2Na, pH 8.5). A solution of the peptide Li5 C dissolved in the coupling buffer (5 mg/mL, 1 mL) was added to the column, and the mixture was allowed to stand at room temperature for 3 hours while gently stirring. After the immobilization of the peptide Li5 C, the bead carrier was washed with distilled water to remove the unreacted peptide, and equilibrated with the coupling buffer. A blocking buffer (a coupling buffer supplemented with 100 mmol/L 2-mercaptoethanol) was added to the equilibrated bead carrier, and the mixture was allowed to stand at room temperature for 3 hours while gently stirring, to perform blocking for avoiding a nonspecific adsorption. After the blocking, the bead carrier was well washed with distilled water to remove unreacted mercaptoethanol. The bead carrier was equilibrated with PBS, and transferred from the column to a limulus test tube with a screw cap (Daiich Pure Chemicals) to be kept at 4°C until use.

According to a measurement with a quantitative reagent for SH group (Ellman's Reagent; PIERCE), 4.9 mg of the peptide Li5 C was immobilized on the bead carrier, and the efficiency of immobilization was 98%. As a control, a bead carrier completely blocked with mercaptoethanol was prepared.

### (3-2) Test for LPS adsorption

LPS prepared from E. coli (E. coli O111:B4; Funakoshi) was dissolved in PBS at a concentration of 1 mg/mL, and the solution was diluted with PBS to prepare an LPS solution (final concentration = 500 ng/mL). From the peptide Li5 C immobilized bead carrier and the control bead carrier prepared in Example 3-1, the dispersing solution (i.e., supernatant) was completely removed. The LPS solution (2 mL) was mixed with each bead carrier, and assay samples (200 µL) taken from the mixtures were transferred to new dry heat sterilized tubes. Immediately, the samples were centrifuged for several seconds using a tabletop centrifuge to precipitate the beads, and supernatants were transferred to new dry heat sterilized tubes. The remaining mixtures after the sample collection were incubated at room temperature while gently stirring, and assay samples were sequentially collected after 30 minutes and 60 minutes in a similar fashion.

Amounts of LPS contained in the collected samples were determined using a commercially available measuring kit (Endospecy ES-50M set; Seikagaku Corporation). With respect to the control (i.e., blocked bead carrier), the LPS concentration of the sample collected immediately after the mixing of the bead carrier with the LPS solution and that of the sample collected after 60 minutes from the mixing were almost the same. In contrast, with respect to the peptide Li5 C immobilized bead carrier, the LPS concentration (A) of the sample collected immediately after the mixing (elapsed time = 0 minute) was 384.7 ng/mL, and that (B) of the sample collected after 30 minutes from the mixing was 85.2 ng/mL. An LPS removal efficiency calculated from the B/A ratio (0.22) was 78%, and a high LPS adsorption was observed.

### (3-3) Evaluation of amounts of peptide immobilized in LPS removal

In this example, bead carriers on which various amounts of the peptide Li5 C were immobilized were prepared, and the LPS removal activities thereof were compared. The procedures described in Example 3-1 were repeated, except that three solutions containing different concentrations (5 mg/mL, 0.5 mg/mL, and 0.05 mg/mL) of the peptide Li5 C were used instead of the solution of the peptide Li5 C (5 mg/mL, 1 mL), to prepare the peptide Li5 C immobilized bead carriers. The result is shown in Table 1. In Table 1, the column A shows the LPS concentrations (unit = ng/mL) of the samples collected immediately after the mixing of the peptide immobilized bead carrier with the LPS solution (elapsed time = 0 minute). The column B shows the LPS concentrations (unit = ng/mL) of the samples collected after 30 minutes from the mixing.

**[Table 1]**

| Immobilized peptide | Amount (mg) | A: 0 min | B: 30 min | Ratio (B/A) | LPS removal (%) |
|---|---|---|---|---|---|
| Li5 C | 5 | 282.4 | 76.8 | 0.27 | 72.8 |
| Li5 C | 0.5 | 282.4 | 219.8 | 0.78 | 22.2 |
| Li5 C | 0.05 | 282.4 | 239.9 | 0.85 | 15.0 |

### (3-4) Evaluation of LPS removal under various conditions

When an LPS and/or lipid A binding agent is used to remove LPS from blood, it is necessary to show the LPS removal activity in the presence of various components suspected of being contained in blood (for example, serum albumin, IL-1β, or heparin), or under various conditions (for example, a concentration of salts or LPS). In this example, it was confirmed that the peptide Li5 C immobilized bead carrier showed a high LPS removal activity under various conditions.

The procedures described in Example 3-2 were repeated except that an LPS solution containing bovine serum albumin (1% BSA, 500 ng/mL LPS) was used instead of the LPS solution (final concentration = 500 ng/mL). As a result, it was found that the presence of BSA did not significantly affect the LPS removal activity of the peptide Li5 C immobilized bead carrier.

The procedures described in Example 3-2 were repeated except that an LPS solution containing human IL-1β (Pepro Tech) (200 ng/mL IL-1β, 500 ng/mL LPS) was used instead of the LPS solution (final concentration = 500 ng/mL). As a result, it was found that the presence of IL-1β did not significantly affect the LPS removal activity of the peptide Li5 C immobilized bead carrier. With respect to IL-1β, nonspecific adsorption was not observed when the peptide Li5 C immobilized bead carrier was used, and this result indicated that LPS could be specifically removed by Li5 C.

When an LPS removal column for extracorporeal circulation is used for removing LPS from blood, an anticoagulant such as heparin or nafamostat mesilate is used. The procedures described in Example 3-2 were repeated except that an LPS solution containing heparin (1 unit/mL heparin, 500 ng/mL LPS) was used instead of the LPS solution (final concentration = 500 ng/mL). As a result, an efficiency of LPS removal in the absence of heparin was 74.4%, and that in the presence of heparin was 45.0%. It was found that the presence of heparin at a commonly-used concentration (1 unit/mL) slightly lowered the LPS removal activity of the peptide Li5 C immobilized bead carrier, but did not significantly affect the activity from a practical point of view.

The procedures described in Example 3-2 were repeated except that an LPS solution containing a salt (NaCl) (0.25, 0.5, or 1 mol/L NaCl, 500 ng/mL LPS) was used instead of the LPS solution (final concentration = 500 ng/mL). The result is shown in Table 2.

**[Table 2]**

| Immobilized peptide | Amount (mg) | [*1] (mol/L) | A: 0 min | B: 30 min | Ratio (B/A) | LPS removal (%) |
|---|---|---|---|---|---|---|
| Li5 C | 5 | 1 | 391.4 | 211.5 | 0.54 | 46.0 |
| Li5 C | 5 | 0.5 | 422.3 | 195.5 | 0.46 | 53.7 |
| Li5 C | 5 | 0.25 | 438.0 | 89.8 | 0.21 | 79.5 |

| | | | | | | |
|---|---|---|---|---|---|---|
| [*1: NaCl concentration in LPS solution] | | | | | | |

In genetic engineering processes for manufacturing useful proteins by Escherichia coli, it is important to remove a high concentration of LPS from a solution of the purified protein. The procedures described in Example 3-2 were repeated except that a phage solution (LPS concentration = 360 µg/mL) was used instead of the LPS solution (final concentration = 500 ng/mL). As a result, it was found that a high concentration of LPS (starting concentration = 360 µg/mL) could be efficiently removed to 30 µg/mL.

Next, solutions containing low concentrations of LPS were used to examine a starting concentration of LPS capable of removing LPS to the pyrogenic threshold of endotoxin, 1 EU/mL. The procedures described in Example 3-2 were repeated except that LPS solutions [LPS (USP Reference Standard Endotoxin; Seikagaku Corporation) concentration = 25 EU/mL, 50 EU/mL, and 100 EU/mL] were used instead of the LPS solution (final concentration = 500 ng/mL). As a result, LPS could be removed from the LPS solution at the starting concentration of 25 EU/mL, to less than 1 EU/mL after 5 minutes. In this connection, the unit EU is an abbreviation for Endotoxin Unit.

### Example 4: Evaluation of various LPS and/or lipid A binding peptides in LPS binding

In this example, various peptides were synthesized, and the LPS binding activities thereof were evaluated to determine which amino acid(s) contributes the LPS binding in the peptide consisting of the following amino acid sequence of SEQ ID NO: 9 determined in Example 1:
KNYSSSISSIHA (SEQ ID NO: 9).
In this connection, peptides synthesized in this example contained cysteine to introduce a thiol group to the C-terminus thereof, unless otherwise specified. Further, any buffers, distilled water, equipment, and the like used in the following procedures were endotoxin-free, unless otherwise specified.

### (4-1) Preparation of LPS and/or lipid A binding peptides

As peptides in which amino acids were sequentially deleted from the C-terminus of the amino acid sequence of SEQ ID NO: 1, the following peptides were synthesized:
Peptide Li5 6C: KNYSSSC (SEQ ID NO: 111)
Peptide Li5 7C: KNYSSSIC (SEQ ID NO: 112)
Peptide Li5 8C: KNYSSSISC (SEQ ID NO: 113)
Peptide Li5 9C: KNYSSSISSC (SEQ ID NO: 114)
Peptide Li5 10C: KNYSSSISSIC (SEQ ID NO: 115)
Peptide Li5 11C: KNYSSSISSIHC (SEQ ID NO: 116)

As a peptide in which the N-terminal amino acid was deleted in the amino acid sequence of SEQ ID NO: 1, the following peptide was synthesized:
Peptide Li5 K1del C: NYSSSISSIHAC (SEQ ID NO: 117)

As peptides in which one or two basic amino acids, i.e., lysine (K) at the first position or histidine (H) at the 11th position, were replaced with different basic amino acids (K, R, or H) in the amino acid sequence of SEQ ID NO: 1, the following peptides were synthesized:
Peptide Li5 H11K C: KNYSSSISSIKAC (SEQ ID NO: 118)
Peptide Li5 H11R C: KNYSSSISSIRAC (SEQ ID NO: 119)
Peptide Li5 K1R H11R C: RNYSSSISSIRAC (SEQ ID NO: 120)
Peptide Li5 K1R C: RNYSSSISSIHAC (SEQ ID NO: 121)
Peptide Li5 K1H C: HNYSSSISSIHAC (SEQ ID NO: 122)

As peptides in which the N-terminal amino acid (L-form) was replaced with a D-form amino acid in the amino acid sequence of SEQ ID NO: 1, the following peptides were synthesized:
Peptide Li5 K1dK C: K (L-form) at the first position in the amino acid sequence of SEQ ID NO: 110 was replaced with D-lysine.
Peptide Li5 K1dR C: K (L-form) at the first position in the amino acid sequence of SEQ ID NO: 110 was replaced with D-arginine.
Peptide Li5 K1dK H11R C: K (L-form) at the first position in the amino acid sequence of SEQ ID NO: 119 was replaced with D-lysine.
Peptide Li5 K1dR H11R C: K (L-form) at the first position in the amino acid sequence of SEQ ID NO: 119 was replaced with D-arginine.

As a peptide in which the N-terminal amino acid was acetylated in the amino acid sequence of SEQ ID NO: 1, the following peptide was synthesized:
Peptide Li5 K1actylK C: K at the first position in the amino acid sequence of SEQ ID NO: 110 was acetylated.

As a peptide in which asparagine (N) at the second position was deleted and histidine (H) at the 11th position was replaced with arginine (R) in the amino acid sequence of SEQ ID NO: 1, the following peptide was synthesized:
Peptide Li5 N2del H10R C: KYSSSISSIRAC (SEQ ID NO: 123). As a peptide in which K (L-form) at the first position was further replaced with D-lysine, the following peptide was synthesized:
Peptide Li5 K1dK N2del H10R C: K (L-form) at the first position was replaced with D-lysine in the amino acid sequence of SEQ ID NO: 123.
As a peptide in which C (L-form) at the 12th position was further replaced with D-cysteine, the following peptide was synthesized:
Peptide Li5 K1dK N2del H10R C12dC: K (L-form) at the first position was replaced with D-lysine and C (L-form) at the 12th position was replaced with D-cysteine in the amino acid sequence of SEQ ID NO: 123.

The following peptides were synthesized:
Peptide Li5 K1dK N2dN H11R C: K (L-form) at the first position was replaced with D-lysine and N (L-form) at the second position was replaced with D-asparagine in the amino acid sequence of SEQ ID NO: 119.
Peptide Li5 -1G K1dK H11R C: K (L-form) at the first position was replaced with D-lysine and G was added to the N-terminus in the amino acid sequence of SEQ ID NO: 119.
Peptide Li5 -lacetylK H11R C: K was added to the N-terminus and the N-terminal K was acetylated, in the amino acid sequence of SEQ ID NO: 119.
Peptide Li5 K1dK H11R C13dC: C (L-form) at the 13th position was replaced with D-cysteine in the amino acid sequence of SEQ ID NO: 119.

### (4-2) Evaluation of LPS binding by BIACORE system

With respect to the peptide Li5 C synthesized in Example 2 and the peptides synthesized in Example 4-1, the BIACORE system (BIACORE 2000; BIACORE) was used to evaluate the LPS binding activities thereof. This evaluation was carried out in accordance with the procedures described in Example 2, except that LPS solutions (concentration = 50 µg/mL, 25 µg/mL, 12.5 µg/mL, and 6.25 µg/mL) were used instead of the lipid A solutions and the LPS solutions (concentration = 100 µg/mL, 50 µg/mL, 25 µg/mL, and 12.5 µg/mL).

### (4-3) Evaluation of LPS adsorption by peptide immobilized beads (by batch method)

With respect to the peptide Li5 C synthesized in Example 2 and the peptides synthesized in **Example 4-1**, the LPS adsorption activities thereof were evaluated by peptide immobilized beads (a batch method). This evaluation (excluding the peptides in which the N at the second position was deleted) was carried out in accordance with the procedures described in Example 3, except that an amount of each peptide to be immobilized on the bead carrier was 2 µmol, 5 mg, or 2 mg. The evaluation of the peptides in which the N at the second position was deleted was carried out in accordance with the procedures described in Example 3, except that 0.2 g (0.5 g in Example 3-1) of the bead carrier, 0.5 mL (1 mL in Example 3-1) of 2-iodoethanol, 50 µL (100 µL in Example 3-1) of pyridine, 2 mg/mL and 1 mL (5 mg/mL and 1 mL in Example 3-1) of the peptide solution, 0.8 mL (2 mL in Example 3-2) of the LPS solution, and 50 µL (200 µL in Example 3-2) of the assay sample were used, and that samples were collected after 5 minutes, 30 minutes, and 60 minutes (after 30 minutes and 60 minutes in Example 3-2).

The results of the peptides in which the C-terminal amino acid(s) were deleted and the peptides in which the basic amino acid(s) were substituted are shown in Table 3 and Table 4, respectively. The result of the peptides in which the N at the second position was deleted are shown in Table 5 and Table 6.

In Table 3 to Table 5, the column A shows the LPS concentrations (unit = ng/mL) of the samples collected immediately after the mixing of the peptide immobilized bead carrier with the LPS solution (elapsed time = 0 minute), and the column B shows the LPS concentrations (unit = ng/mL) of the samples collected after 30 minutes from the mixing. In Table 6, the column A shows the LPS concentrations (unit = ng/mL) of the samples collected immediately after the mixing of the peptide immobilized bead carrier with the LPS solution (elapsed time = 0 minute), and the column B shows the LPS concentrations (unit = ng/mL) of the samples collected after 5 minutes from the mixing.

**[Table 3]**

| Immobilized peptide | Amount (µmol) | A: 0 min | B: 30 min | Ratio (B/A) | LPS removal (%) |
|---|---|---|---|---|---|
| Li5 Kldel C | 2 | 277.0 | 166.8 | 0.60 | 39.8 |
| Li5 11C | 2 | 277.0 | -12 | 0 | 100.0 |
| Li5 10C | 2 | 277.0 | 67.7 | 0.24 | 75.6 |
| Li5 9C | 2 | 277.0 | 202.8 | 0.73 | 26.8 |
| Li5 8C | 2 | 277.0 | 230.3 | 0.83 | 16.9 |
| Li5 7C | 2 | 277.0 | 242.5 | 0.88 | 12.5 |
| Li5 6C | 2 | 277.0 | 234.1 | 0.85 | 15.5 |

**[Table 4]**

| Immobilized peptide | Amount (mg) | A: 0 min | B: 30 min | Ratio (B/A) | LPS removal (%) |
|---|---|---|---|---|---|
| Li5 C | 5 | 458.4 | 148.4 | 0.32 | 67.6 |
| Li5 K1dK C | 5 | 458.4 | 202.0 | 0.44 | 55.9 |
| Li5 K1 actylKC | 5 | 458.4 | 215.7 | 0.47 | 52.9 |
| Li5 K1R C | 5 | 458.4 | 53.0 | 0.12 | 88.4 |
| Li5 K1H C | 5 | 458.4 | 158.9 | 0.35 | 65.3 |
| Li5 H11R C | 5 | 458.4 | 78.3 | 0.17 | 82.9 |
| Li5 H11K C | 5 | 458.4 | 128.6 | 0.28 | 71.9 |
| Li5 K1R H11RC | 5 | 372.0 | 55.6 | 0.15 | 85.1 |
| Li5 K1dR C | 5 | 457.3 | 185.8 | 0.41 | 59.4 |
| Li5 K1dR H11RC | 5 | 457.3 | 195.4 | 0.43 | 57.3 |

**[Table 5]**

| Immobilized peptide | Amount (mg) | A: 0 min | B: 30 min | Ratio (B/A) | LPS removal (%) |
|---|---|---|---|---|---|
| Li5 N2del H10R C | 2 | 405.5 | 16.2 | 0.04 | 96.0 |
| Li5 K1dK N2del H10R C | 2 | 405.5 | 19.3 | 0.05 | 95.3 |
| Li5 K1dK N2del H10R C12dC | 2 | 407.6 | 29.6 | 0.07 | 92.7 |

**[Table 6]**

| Immobilized peptide | Amount (mg) | A: 0 min | B: 30 min | Ratio (B/A) | LPS removal (%) |
|---|---|---|---|---|---|
| Li5 N2del H10R C | 2 | 405.5 | 136.4 | 0.35 | 66.4 |
| Li5 K1dK N2del H10R C | 2 | 405.5 | 36.7 | 0.09 | 91.0 |
| Li5 K1dK N2del H10R C12dC | 2 | 407.6 | 114.7 | 0.28 | 71.9 |

### (4-4) Results

The results obtained in Example 4-2 and Example 4-3, as well as a result of stability in blood, are shown in Table 7. The peptides used are summarized in Table 8.

The stability in blood was evaluated in accordance with the following procedure. Each peptide was dissolved in distilled water at a concentration of 10 mg/mL, and the solutions were diluted to 1/10 with distilled water to adjust a final concentration to 1 mg/mL. Each peptide solution (10 µL) was added to human blood plasma (50 µL) and, after 5 minutes, 30% TCA (25 µL) was added to stop the reaction.

Each mixture was stirred and centrifuged at 12,000 rpm for 5 minutes to precipitate denatured proteins. Each supernatant was applied to high performance liquid chromatography (HPLC) under the following conditions:
Column: Tosoh ODS 80TM (4.6 × 100 mm)
Solvent A: 0.1% trifluoroacetic acid (TFA)
Solvent B: 90% acetonitrile (in 0.1% TFA)
Flow rate: 0.8 mL/min
Gradient: 10 to 60% B (for 55 minutes)
Monitoring: Absorbance at 210 nm (1.0 AUSF) and 280 nm (0.2 AUSF)
The above procedure [i.e., 30% TCA (25 µL) was added to stop the reaction, denatured proteins were precipitated by centrifugation, and each supernatant was analyzed by HPLC] was repeated, and the height of each peak was measured. Further, the amino acid sequence of each peak was determined using a sequencer, to confirm degraded products and evaluate the stability in blood plasma.

In Table 7, the column (a) shows a binding force (KD), the column (b) shows an amount of LPS bound (Rmax) in the case of 50 µg/mL LPS (calculated by a BIACORE software), the column (c) shows an evaluation by the BIACORE system [i.e., an evaluation based on the columns (a) and (b)], the column (d) shows an evaluation by the batch method, the column (e) shows an evaluation with respect to the stability in blood, and the column (f) shows a total evaluation [i.e., evaluation based on the columns (c) to (e)].

The evaluations in the columns (c) to (f) are graded from A to D. The level A means excellent, the level B means good, the level C means practically available, and the level D means poor when used alone, but available by an appropriate modification, for example, by a use as a multimer consisting of a repetitive sequence, or by a high-density immobilization of the peptide.

In the evaluation by the BIACORE system of the column (c) [i.e., the evaluation based on the columns (a) and (b)], when the values shown in the columns (a) and (b) are totally evaluated in comparison with those of the peptide Li5 C, a peptide superior to the peptide Li5 C is regarded as the level A, a peptide similar to the peptide Li5 C is regarded as the level B, a peptide slightly inferior to the peptide Li5 C is regarded as the level C, and a peptide inferior to the peptide Li5 C is regarded as the level D.

In the evaluation by the batch method of the column (d), a peptide showing an LPS adsorption superior to that of the peptide Li5 C or an efficiency of LPS removal of 70% or more is regarded as the level A, a peptide showing an LPS adsorption similar to that of the peptide Li5 C or an efficiency of LPS removal of 50% to 69% is regarded as the level B, a peptide showing an LPS adsorption slightly inferior to that of the peptide Li5 C or an efficiency of LPS removal of 30% to 49% is regarded as the level C, and a peptide showing an LPS adsorption inferior to that of the peptide Li5 C or an efficiency of LPS removal of 10% to 29% is regarded as the level D.

In the evaluation with respect to the stability in blood of the column (e), a peptide which remains in blood plasma over 1 hour is regarded as the level A, a peptide which is degraded in blood plasma in 30 minutes to 1 hour is regarded as the level B, a peptide which is completely degraded in blood plasma in 10 minutes to 30 minutes is regarded as the level C, and a peptide which is completely degraded in blood plasma in 1 minute to 10 minutes is regarded as the level D.

These results show it is preferable that the N-terminal amino acid is a basic amino acid, particularly K. This is supported by the result that the LPS binding activity was lowered by deleting the N-terminal K. It is preferable that the amino group at the N-terminus is free, because the binding force was weakened by acetylating the N-terminal amino group. It is preferable that an amino acid at the 11th or 10th position from the N-terminus is a basic amino acid, particularly R. This is supported by the result that when one or plural amino acids are deleted from the C-terminus, the LPS binding activity was lowered by deleting a basic amino acid at the above position. It is preferable from the viewpoint of stability that the N-terminal amino acid is a D-form. In addition, it is synergistically effective in developing the stability that the C-terminal amino acid is a D-form. It was found from the results of the peptides in which the N at the second position was deleted that the efficiency of LPS adsorption was increased by replacing the N-terminal K with D-form K. Further, the LPS adsorption in the peptides in which the amino acids at both terminals were D-forms was confirmed. The result indicates that the adsorption activity is not lowered by replacing the N-terminal amino acid with a D-form, or by replacing the amino acids at both terminals with D-forms.

**[Table 7]**

| Peptide | (a) | (b) | (c) | (d) | (e) | (f) |
|---|---|---|---|---|---|---|
| Li5 C | 10⁻⁷-10⁻⁸ | 2590 | B | B | C | B |
| Li5 K1R C | 10⁻⁸(10⁻⁹) | 1390 | B | B | C | B |
| Li5 K1H C | 10⁻¹¹(10⁻¹⁰) | 76.9 | B | B | - | B |
| Li5 H11R C | 10⁻⁷-10⁻⁹ | 2380 | A | A | - | A |
| Li5 H11K C | 10⁻⁷-10⁻⁹ | 2440 | A | B | - | B |
| Li5 K1R H11R C | 20⁻⁸(10⁻⁹) | 699 | B | A | C | A |
| Li5 K1dK C | 10⁻¹⁰-10⁻¹¹ | 886 | B | A | A | A |
| Li5 K1dK H11R C | - | - | - | A | A | A |
| Li5 K1dR C | 10⁻⁸ | 221 | C | B | A | B |
| Li5 K1dR H11R C | 10⁻⁸(10⁻¹²) | 195 | C | B | A | B |
| Li5 K1dK N2dN H11R C | - | - | - | B | A | A |
| Li5 -1G K1dK H11R C | - | - | - | B | A | A |
| Li5 KlactylK C | 10⁻⁹(10⁻¹⁰) | 210 | B | C | A | B |
| Li5 -lacetylK H11R C | - | - | - | A | A | A |
| Li5 K1dK H11R C13dC | - | - | - | A | A | A |
| Li5 N2del H10R C | - | - | - | A | B | A |
| Li5 K1dK N2del H10R | 1o⁻¹⁰-10⁻¹¹ | 6140 | A | A | A | A |
| Li5 K1dK N2del H10R C12dC | 10⁻¹⁰-10⁻¹¹ | 4290 | A | A | A | A |
| Li5 K1del C | 10⁻⁸-10⁻⁹ | 452 | B | C | - | C |
| Li5 11C | 10⁻⁸-10⁻⁹(10⁻¹¹) | 1240 | A | A | - | B |
| Li5 10C | - | - | - | A | - | B |
| Li5 9C | 10⁻⁷-10⁻⁸ | 585 | C | C | - | C |
| Li5 8C | - | - | - | D | - | D |
| Li5 7C | 10⁻⁸ | 58.4 | C | D | - | D |
| Li5 6C | - | - | - | D | - | D |

**[Table 8]**

| Peptide | Amino acid | sequence |
|---|---|---|
| Li5 C | KNYSSSISSIHAC | (SEQ ID NO: 110) |
| Li5 K1R C | RNYSSSISSIHAC | (SEQ ID NO: 121) |
| Li5 K1H C | HNYSSSISSIHAC | (SEQ ID NO: 122) |
| Li5 H11R C | KNYSSSISSIRAC | (SEQ ID NO: 119) |
| Li5 H11K C | KNYSSSISSIKAC | (SEQ ID NO: 118) |
| Li5 K1R H11R C | RNYSSSISSIRAC | (SEQ ID NO: 120) |
| Li5 K1dK C | kNYSSSISSIHAC | |
| Li5 K1dK H11R C | kNYSSSISSIRAC | |
| Li5 K1dR C | rNYSSSISSIHAC | |
| Li5 K1dR H11R C | rNYSSSISSIRAC | |
| Li5 K1dK N2dN H11R C | knYSSSISSIRAC | |
| Li5 -1G K1dK H11R C | GkNYSSSISSIRAC | |
| Li5 K1actylK C | aKNYSSSISSIHAC | |
| Li5 -1acetylK H11R C | aKKNYSSSISSIRAC | |
| Li5 K1dK H11R C13d C | kNYSSSISSIRAc | |
| Li5 N2del H10R C | K YSSSISSIRAC | (SEQ ID NO: 123) |
| Li5 K1dK N2del H10R C | k YSSSISSIRAC | |
| Li5 K1dK N2del H10R C12dC | k YSSSISSIRAc | |
| Li5 K1del C | NYSSSISSIHAC | (SEQ ID NO: 117) |
| Li5 11C | KNYSSSISSIH C | (SEQ ID NO: 116) |
| Li5 10C | KNYSSSISSI C | (SEQ ID NO: 115) |
| Li5 9C | KNYSSSISS C | (SEQ ID NO: 114) |
| Li5 8C | KNYSSSIS C | (SEQ ID NO: 113) |
| Li5 7C | KNYSSSI C | (SEQ ID NO: 112) |
| Li5 6C | KNYSSS C | (SEQ ID NO: 111) |

In Table 8, the amino acids written in lowercase letters (k, r, n, and c) are D-form amino acids, and the abbreviation ak means that the N-terminal amino group is acetylated.

### Example 5: LPS removal by open column method

### (5-1) Immobilization of peptide Li5 C on bead carrier

In this example, a reaction was carried out by adding a solvent to silica gel with acid chloride (Propionyl chloride functionalized silica gel 200-400 mesh; Sigma-Aldrich)(hereinafter referred to as bead carrier) to immobilize the peptide Li5 C on the bead carrier via the thiol group of the C-terminal cysteine of the peptide Li5 C. In this connection, any buffers, distilled water, equipment, and the like used in the following procedures were endotoxin-free, unless otherwise specified.

More particularly, the bead carrier (1.0 g) was weighed out and put into a dry heat sterilized test tube. To the test tube, 2-iodoethanol (Wako Pure Chemicals; 2 mL) was added to react the hydroxyl group of iodoethanol with acid chloride conjugated to the bead carrier, to form a covalent bond. In this reaction, hydrogen chloride (HCl) would be produced as a by-product. Pyridine (Wako Pure Chemicals; a compound containing amine base; 200 µL) was added to this reaction system to avoid the side reaction of iodoethanol. The test tube was covered with parafilm and the whole was mixed well. The reaction was carried out at room temperature for 4 hours while gently stirring.

The reaction products were transferred to an Econo-Column (catalog No. 737-0516, iInternal diameter = 0.5 cm, length = 15 cm, area of bottom = 0.2 cm²; BIO-RAD), well washed with distilled water, and equilibrated with a coupling buffer (50 mmol/L Tris-HCl, 5 mmol/L EDTA·2Na, pH 8.5). A solution of the peptide Li5 C dissolved in the coupling buffer (2.5 mg/mL, 2 mL) was added to the column, and the mixture was allowed to stand at room temperature for 3 hours while gently stirring. After the immobilization of the peptide Li5 C, the bead carrier was washed with distilled water to remove the unreacted peptide, and equilibrated with the coupling buffer. A blocking buffer (a coupling buffer supplemented with 100 mmol/L 2-mercaptoethanol) was added to the equilibrated bead carrier, and the mixture was allowed to stand at room temperature for 3 hours while gently stirring, to perform blocking for avoiding a nonspecific adsorption. After the blocking, the bead carrier was well washed with distilled water to remove unreacted mercaptoethanol. The bead carrier was equilibrated with PBS, and the top and the bottom of the column were capped to be kept at 4°C until use.

According to a measurement with a quantitative reagent for SH group (Ellman's Reagent; PIERCE), 4.95 mg of the peptide Li5 C was immobilized on the bead carrier, and the efficiency of immobilization was 99%. As a control, a bead carrier completely blocked with mercaptoethanol was prepared.

### (5-2) Test for LPS removal

LPS prepared from E. coli (E. coli O111:B4; Funakoshi) was dissolved in PBS at a concentration of 1 mg/mL, and the solution was diluted with PBS to prepare an LPS solution (final concentration = 500 ng/mL). The solution (PBS) was removed from the column prepared in Example 5-1, up to the height of the top of the bead carrier. The LPS solution (10 mL) was applied from the top of the column, and LPS eluted from the bottom of the column was sequentially collected with a volume of 0.5 mL in dry heat sterilized tubes. Amounts of LPS contained in the collected samples were determined in accordance with the method described in Example 3-2. As a result, although the control column was saturated after a while, the LPS removal could be efficiently carried out with the peptide Li5 C immobilized column (LPS removal efficiency = 91.1%).

### Example 6: LPS assay using peptide immobilized bead carrier

In this example, a bead carrier on which the peptide Li5 K1dK N2del H10R C12dC synthesized in Example 4-1 was immobilized was reacted with LPS, and an amount of LPS bound to the bead carrier was measured, to confirm that LPS can be determined using the peptide immobilized bead carrier. For comparison, a bead carrier completely blocked with mercaptoethanol was prepared as a control. In this connection, any buffers, distilled water, equipment, and the like used in the following procedures were endotoxin-free, unless otherwise specified.

### (6-1) Immobilization of peptide Li5 K1dK N2del H10R C12dC on bead carrier

In this example, a reaction was carried out by adding a solvent to silica gel with acid chloride (Propionyl chloride functionalized silica gel 200-400 mesh; Sigma-Aldrich) (hereinafter referred to as bead carrier) to immobilize the peptide Li5 C on the bead carrier via the thiol group of the C-terminal cysteine of the peptide.

More particularly, the bead carrier (10 mg per sample) was weighed out and put into a dry heat sterilized test tube. To the test tube, 2-iodoethanol (Wako Pure Chemicals; 0.1 mL) was added to react the hydroxyl group of iodoethanol with acid chloride conjugated to the bead carrier, to form a covalent bond. In this reaction, hydrogen chloride (HCl) would be produced as a by-product. Pyridine (Wako Pure Chemicals; a compound containing amine base; 10 µL) was added to this reaction system to avoid the side reaction of iodoethanol. The test tube was covered with parafilm and the whole was mixed well. The reaction was carried out at room temperature for 4 hours while gently stirring.

The reaction products were transferred to an Econo-Column (BIO-RAD), well washed with distilled water, and equilibrated with a coupling buffer (50 mmol/L Tris-HCl, 5 mmol/L EDTA·2Na, pH 8.5). A solution of the peptide dissolved in the coupling buffer (20 mg/mL, 0.25 mL) was added to the column, and the mixture was allowed to stand at room temperature for 3 hours while gently stirring. After the immobilization of the peptide, the bead carrier was washed with distilled water to remove the unreacted peptide, and equilibrated with the coupling buffer. A blocking buffer (a coupling buffer supplemented with 100 mmol/L 2-mercaptoethanol) was added to the equilibrated bead carrier, and the mixture was allowed to stand at room temperature for 3 hours while gently stirring, to perform blocking for avoiding a nonspecific adsorption. After the blocking, the bead carrier was well washed with distilled water to remove unreacted mercaptoethanol. The bead carrier was equilibrated with PBS, and transferred from the column to a dry heat sterilized tube (Seikagaku Corporation) to be kept at 4°C until use.

An amount of the peptide immobilized on the bead carrier was determined with a measurement with a quantitative reagent for SH group (Ellman's Reagent; PIERCE), and the efficiency of immobilization was 90% or more. As a control, a bead carrier completely blocked with mercaptoethanol was prepared.

### (6-2) Test for LPS adsorption

An endotoxin standard [Reference Standard Endotoxin (RSE); Seikagaku Corporation] prepared from E. coli O113:H10 strain in the United States Pharmacopeia was dissolved in endotoxin-free distilled water [LAL Reagent Water (LRW); Seikagaku Corporation] at a concentration of 2,000 EU/mL, and the solution was diluted with PBS to prepare various concentrations of LPS solutions (final concentration = 10 EU/mL, 5 EU/mL, 2.5 EU/mL, and 0 EU/mL). From the peptide immobilized bead carrier and the control bead carrier prepared in Example 6-1 in the test tubes, the dispersing solution (i.e., supernatant) was completely removed. Each (1 mL) of the various concentrations of LPS solutions was mixed with each bead carrier in the test tube. After supernatants (10 µL) were taken from the mixtures as assay samples immediately after the mixing, the test tubes were covered with parafilm and a reaction was carried out at room temperature for 30 minutes while gently stirring. The mixtures were centrifuged for several seconds using a tabletop centrifuge to precipitate the beads, and supernatants were transferred to new dry heat sterilized tubes as supernatants after the reaction. To the precipitated beads, PBS (0.5 mL) was added, and the washing treatment consisting of mixing, the centrifugation, and the supernatant removal was repeated six times (total volume = 3 mL) to remove unbound LPS. The solutions obtained by the washing treatment were kept in dry heat sterilized tubes to determine LPS concentrations.

To the remaining bead carriers, PBS (1 mL) was added and mixed. The supernatants taken immediately after the mixing, the supernatants taken after the reaction, and the solutions obtained by the washing treatment were subjected to an LPS assay. An amount of LPS was determined using a commercially available measuring kit (Endospecy ES-50M set; Seikagaku Corporation). The result is shown in Table 9.

**[Table 9]**

| | [*1] | [*2] | [*3] | [*4] | [*5] |
|---|---|---|---|---|---|
| Control | EU/mL | EU | EU | EU | EU |
| | 0 | -0.05 | 0.00 | 0.00 | -0.01 |
| | 2.5 | -0.04 | 2.61 | 3.10 | 0.08 |
| | 5 | 0.01 | 5.16 | 5.32 | 0.10 |
| | 10 | 0.08 | 10.20 | 12.18 | 0.07 |

| | EU/mL | EU | EU | EU | EU |
|---|---|---|---|---|---|
| Li5 K1dK N2del H10R C12dC | 0 | 0.85 | 0.19 | 0.11 | -0.04 |
| | 2.5 | 4.16 | 2.53 | -0.28 | 0.06 |
| | 5 | 6.20 | 4.74 | -0.67 | 0.21 |
| | 10 | 9.85 | 9.55 | -0.48 | 0.15 |

| | | | | | |
|---|---|---|---|---|---|
| [*1: Amount of LPS added] [*2: Amount of LPS bound to beads] [*3: Amount of LPS immediately after the mixing] [*4: Amount of LPS contained in supernatant after the reaction] [*5: Amount of LPS contained in the solution obtained by the washing treatment] | | | | | |

As a result, amounts of LPS bound to the control bead carrier were approximately 0 EU, and amounts of LPS bound to the peptide immobilized bead carrier could be determined concentration-dependently. Amounts of LPS contained in the supernatants taken immediately after the LPS addition accorded with those added; the solutions obtained after the washing contained little LPS (0 EU); and little LPS remained in the supernatants taken after the reaction. These results indicate LPS bound to a bead carrier can be accurately determined.

### Example 7: Confirmation of efficiency of LPS removal by open column method

In this example, a column filled with a peptide Li5 K1dK N2del H10R C12dC immobilized carrier was compared to that filled with a non-immobilized carrier, with respect to the LPS removal efficiency. In this connection, any buffers, distilled water, equipment, and the like used in the following procedures were endotoxin-free, unless otherwise specified.

### (7-1) Immobilization of peptide on bead carrier

In this example, a carrier capable of immobilizing a substance having a thiol group(s) via a disulfide (S-S) bond (Thiopropyl Sepharose 6B Lab Pack; Amersham Biosceiences) (hereinafter referred to as carrier) was used to immobilize the peptide on the carrier.

More particularly, the carrier [0.5 g (volume = 1.5 mL)] was weighed out and put into a centrifuge tube. Distilled water was added to the tube, and the swollen carrier was transferred to an Econo-Column (BIO-RAD) (hereinafter referred to as column). Distilled water (100 mL) was applied to the column to wash the carrier. A peptide solution, which had been prepared by dissolving 5 mg of the peptide in 1.35 mL of distilled water and 0.15 mL of 0.1 mol/L formic acid (pH 4.5), was added to the column, and the column was allowed to stand at room temperature overnight to immobilize the peptide on the carrier. A blocking buffer [a solution mixture (distilled water : 0.1 mol/L formic acid (pH 4.5) = 9:1) supplemented with 100 mmol/L 2-mercaptoethanol] was added to the column, and the column was allowed at room temperature overnight to perform blocking for avoiding a nonspecific adsorption. After the blocking, distilled water was applied to the column to remove unreacted mercaptoethanol. The column was equilibrated with PBS and kept at 4°C until use.

For a control, a carrier completely blocked with mercaptoethanol was prepared.

### (7-2) Confirmation test for LPS removal

The PBS solution was removed form the column, up to the height of the top of the carrier. To the column, 8 mL of an LPS solution (10 ng/mL LPS E.coli O111:B4 dissolved in human blood plasma) was applied, and LPS eluted from the bottom of the column was sequentially collected with a volume of 1 mL or 0.5 mL. The collected samples were stocked in dry heat sterilized tubes.

Amounts of LPS contained in the samples were determined to confirm the efficiency of the peptide in LPS removal. The amounts of LPS were determined using a commercially available measuring kit (Endospecy ES-50M set; Seikagaku Corporation).

As a result, the control column (carrier alone) was saturated immediately after the PBS solution was eluted. In contrast, the peptide immobilized column maintained an LPS removal efficiency of approximately 50% for a while, and gradually saturated. After 8 mL of the LPS solution was eluted, the removal efficiency of the peptide immobilized column did not reach a completely saturated state. This result shows that the peptide immobilized carrier can be used to remove LPS from blood plasma.

### Example 8: Evaluation of peptide in stability

In this example, the stability of the peptide Li5 K1dK N2del H10R C12dC was measured using the BIACORE system (BIACORE 2000; BIACORE) for measuring a strength of binding between a protein and a molecule such as a protein or an amount of binding therebetween. For comparison, polymyxin B (PMB) was measured. In this connection, any buffers, distilled water, equipment, and the like used in the following procedures were endotoxin-free, unless otherwise specified.

The peptide Li5 K1dK N2del H10R C12dC was immobilized on a flow cell (Fc), Fc2, of a sensor chip (BIACORE Sensor Chip CM5 ; BIACORE) by thiol coupling. As a control, cysteine was immobilized on Fc1. For comparison, PMB was immobilized on Fc4 via an amino group by amine coupling. In this connection, equimolar substances were immobilized.

A 50 µg/mL LPS solution [Buffer HBS-EP (0.01 mol/L HEPES, pH7.4, 0.15 mol/L NaCl, and 3 mmol/L EDTA)] was applied to the sensor chip as an analyte to measure an amount of binding (RU: Resonance Unit), and a strong alkaline solution (15 mmol/L NaOH) was applied to wash the sensor chip. This cycle was repeated to evaluate the stability of the peptide on the basis of the change in the amount of binding.

As a result, while the amount of LPS binding to PMB was decreased dependently on the cycle number, the amount of LPS binding to the peptide Li5 K1dK N2del H10R C12dC did not change and remained constant. This result indicated that the peptide was stable to a strong alkaline washing.

### Example 9: Fluorescent staining of Gram-negative bacteria

In this example, the peptide Li5 K1dK N2del H10R C synthesized in Example 4-1 was used to perform fluorescent staining of Gram-negative bacteria (E. coli and Pseudomonas aeruginosa) and Gram-positive bacteria (Lactobacillus).

To 1 mL of a solution of the peptide Li5 K1dK N2del H10R C (0.5 mg/mL) in PBS (pH7.2), 5 mg of fluorescein-5-maleimide (PIERCE) was added according to a conventional method. After a reaction at room temperature for 2 hours, blocking was performed by adding 50 mg of L-cysteine thereto, to prepare a fluorescein-labeled peptide. The fully grown bacteria were independently collected by centrifugation, and resuspended in a volume of water ten times as much as the volume of the culture medium before the centrifugation. An aliquot (5 µL) of each bacterial suspension was dropped on a silane-treated slide glass, and the slide glass was dried. To fix the bacteria on the slide glass, 10 µL of 100% ethanol was dropped thereon, and the slide glass was dried. Then, 100 µL of the fluorescein-labeled peptide solution (25 µg/mL) was dropped thereon, and the slide glass was allowed to stand for 3 hours to carry out the staining. As a control, a fluorescein-labeled cysteine solution or water was used instead of the fluorescein-labeled peptide solution.

As a result of an observation with a fluorescence microscope (BX50WI; Olympus), the Gram-negative bacteria, E.coli and Pseudomonas aeruginosa, were stained with the fluorescein-labeled peptide, but the Gram-positive bacteria, Lactobacillus, were not stained with the same. Neither the Gram-negative bacteria nor the Gram-positive bacteria were stained with the fluorescein-labeled cysteine used as a control.

### Example 10: Evaluation of binding-force (affinity) of Li5-025 to LPS derived from various bacteria (by BIACORE system)

In this example, the BIACORE system (BIACORE 2000; BIACORE) was used to evaluate the binding force of the peptide Li5 K1dK N2del H10R C synthesized in Example 4-1 (hereinafter sometimes referred to as peptide Li5-025) to LPS prepared from various bacteria different in species.

More particularly, the synthesized peptide Li5-025 was immobilized on a flow cell (hereinafter referred to as Fc), Fc2, of a sensor chip (BIACORE Sensor Chip CM5 ; BIACORE) by thiol coupling. As a control, cysteine was immobilized on Fc1.

With respect to each LPS from various bacteria [Bordetella pertussis (Bp), Vibrio cholerae serotype Inaba 569B (Vc), Klebsiella pneumoniae (Kp), Pseudomonas aeruginosa Serotype 10 (Pa), and Escherichia coli O111:B4 (Ec)], various concentrations of LPS solutions [concentration = 50 µg/mL, 25 µg/mL, 12.5 µg/mL, and 6.25 µg/mL; Buffer HBS-EP (Tween-20 free) (0.01 mol/L HEPES, pH 7.4, 0.15 mol/L NaCl, and 3 mmol/L EDTA)] were applied as an analyte to the sensor chip in order of an increasing concentration to carry out the measurement.

As a result, the binding force [average of a dissociation constant (KD)] of the peptide to the LPS derived from Bp, Kp, or Ec was 10⁻⁸, that to the LPS derived from Vc was 10⁻⁸ to 10⁻⁹, and that to the LPS derived from Pa was 10⁻¹¹ to 10⁻¹². It was confirmed that the peptide Li5-025 could strongly bind to each LPS derived from these bacteria and each Kd was of the order of 10⁻⁸.

### Example 11: Evaluation of Li5-025 in various LPS adsorptions (by batch method)

In this example, the peptide Li5-025 and each LPS used in Example 10 were used to measure the adsorption activity of the peptide to each LPS by a batch method, as well as that of polymyxin B (PMB) for comparison. In this connection, any buffers, distilled water, equipment, and the like used in the following procedures were endotoxin-free, unless otherwise specified.

### (1) Immobilization of peptide

A reaction was carried out by adding a solvent to silica gel with acid chloride (Propionyl chloride functionalized silica gel 200-400 mesh; Sigma-Aldrich) (hereinafter referred to as bead carrier) to immobilize the peptide Li5-025 on the bead carrier by a covalent bond via the thiol group of the C-terminal cysteine of the peptide Li5-025.

More particularly, the bead carrier (0.2 g) was weighed out and put into a dry heat sterilized test tube. To the test tube, 2-iodoethanol (Wako Pure Chemicals; 0.4 mL) was added to react the hydroxyl group of iodoethanol with acid chloride conjugated to the bead carrier, to form a covalent bond. In this reaction, hydrogen chloride (HCl) would be produced as a by-product. Pyridine (Wako Pure Chemicals; a compound containing amine base; 40 µL) was added to this reaction system to avoid the side reaction of iodoethanol. The test tube was covered with parafilm and the whole was mixed well. The reaction was carried out at room temperature for 4 hours while gently stirring.

The reaction products were transferred to an Econo-Column (BIO-RAD), well washed with distilled water, and equilibrated with a coupling buffer (50 mmol/L Tris-HCl, 5 mmol/L EDTA·2Na, pH 8.5). A solution of the peptide Li5-025 dissolved in the coupling buffer (2 mg/mL, 1 mL) was added to the column, and the mixture was allowed to stand at room temperature for 6 hours while gently stirring. A solution was removed from the mixture after the peptide immobilization, and was used to determine an amount of the peptide immobilized, using a quantitative reagent for SH group (Ellman's Reagent; PIERCE).

After the immobilization of the peptide Li5-025, the bead carrier was washed with distilled water to remove the unreacted peptide, and equilibrated with the coupling buffer. A blocking buffer [a coupling buffer supplemented with 100 mmol/L 2-mercaptoethanol (Kanto Chemical)] was added to the equilibrated bead carrier, and the mixture was allowed to stand at room temperature for 3 hours while gently stirring, to perform blocking for avoiding a nonspecific adsorption.

After the blocking, the bead carrier was well washed with distilled water to remove unreacted mercaptoethanol. The bead carrier was equilibrated with PBS, and transferred from the column to a limulus test tube with a screw cap (Daiich Pure Chemicals) to be kept at 4°C until use.

### (2) Immobilization of polymyxin B (PMB)

In this example, a reaction was carried out by adding a solvent to silica gel with acid chloride (Propionyl chloride functionalized silica gel 200-400 mesh; Sigma-Aldrich)(hereinafter referred to as bead carrier) to immobilize PMB on the bead carrier by a covalent bond via an amino group.

More particularly, the bead carrier (0.2 g) was weighed out and put into a dry heat sterilized test tube. Distilled water (H₂O) was added to the test tube, and the tube was converted with parafilm and allowed to stand at room temperature with gently stirring until bubbles disappeared, to react water with the acid chloride conjugated to the bead carrier and replace the chloride portion with a hydroxyl group (-OH) by a covalent bond.

After the disappearance of bubbles in the test tube was confirmed, the bead carrier was transferred to an Econo-Column (BIO-RAD), washed with distilled water, and equilibrated with a 0.1 mol/L NaHCO₃ buffer (pH 8.0). EDC [1-Ethyl-3-(3-dimethylaminopropyl)carbodiimide hydrochloride] and NHS (N-hydroxysuccinimide) were added to the column, and the reaction was carried out at room temperature for 1 hour. After the reaction, the solution in the column was drained, and washed with a 0.1 mol/L NaHCO₃ buffer. To the column, a PMB solution (2 mg/mL, 1 mL) dissolved in a 0.1 mol/L NaHCO₃ buffer was added, and the whole was allowed to stand at room temperature for 6 hours with gently stirring. A solution was removed from the mixture after the PMB immobilization, and was used to determine an amount of PMB immobilized, using a quantitative kit for amino group (CBQCA Protein Quantitation Kit; Molecular Probes).

After the immobilization of PMB, the bead carrier was washed with distilled water to remove unreacted PMB, and equilibrated with a 0.1 mol/L NaHCO₃ buffer. A blocking buffer was prepared by dissolving 1.0 mol/L ethanolamine (Wako Pure Chemicals) in distilled water and adjusting pH to 8.5 with HCl. The blocking buffer (3 mL) was added to the bead carrier, and the mixture was allowed to stand at room temperature for 3 hours while gently stirring, to perform blocking for avoiding a nonspecific adsorption.

After the blocking, the bead carrier was well washed with distilled water to remove unreacted ethanolamine. The bead carrier was equilibrated with PBS, and transferred from the column to a limulus test tube with a screw cap (Daiich Pure Chemicals) to be kept at 4°C until use.

### (3) Preparation of control (non-immobilized bead carrier)

As a control, only the blocking treatment described in Example 11(1) was carried out to prepare a bead carrier completely blocked with mercaptoethanol.

### (4) Test for LPS (endotoxin) adsorption and method of collecting samples

LPS solutions were prepared, and independently mixed with the peptide (or PMB) immobilized bead carriers, and samples for measurement were sequentially collected in accordance with the following procedures.

More particularly, the dispersing solution (i.e., supernatant) was completely removed from immobilized or blocking bead carriers. Each LPS was dissolved in PBS and adjusted to a concentration of 500 ng/mL to prepare LPS solutions. An aliquot (0.8 mL) of each LPS solution was mixed with each bead carrier, and samples (200 µL) were collected and transferred to new dry heat sterilized tubes. Immediately, the samples were centrifuged for several seconds using a tabletop centrifuge to precipitate the beads, and supernatants were transferred to new dry heat sterilized tubes. The remaining bead suspensions after the sample collection were well mixed and incubated at room temperature while gently stirring. Assay samples were sequentially collected in a similar fashion.

### (5) Determination of amount of endotoxin

Amounts of endotoxin contained in the collected samples were measured using a commercially available measuring kit (Endospecy ES-50M set; Seikagaku Corporation) to confirm the LPS adsorption activity in accordance with the following procedures.

The collected samples were diluted with distilled water so as to fall within the measuring range of the kit. Diluted samples (50 µl/well) were added to wells of a 96-well polystylene Toxipet plate (Et Free; Seikagaku Corporation). The Endospecy ES-50M set was prepared, and the whole amount of a buffer contained in the kit was added to a lysate reagent (a tube) contained in the kit, to dissolve it by shaking without foaming for 5 minutes. The solution (50 µl/well) was added to the wells containing the diluted samples, and mixed well. The plate was incubated at 37°C for 30 minutes while gently stirring.

An absorbance at 405 nm was measured with an absorptiometer (ARVO; wallac). A calibration curve was prepared, and LPS concentrations were calculated from an equation of approximate line.

The result is shown in Table 10. The abbreviations Bp, Vc, Kp, and Ec in Table 10 denote Bordetella pertussis, Vibrio cholerae serotype Inaba 569B, Klebsiella pneumoniae, and Escherichia coli O111:B4, respectively.

With respect to the control, the concentrations of LPS taken immediately after the mixing and taken after 60 minutes did not change and remained fairly constant. In contrast, with respect to the Li5-025 immobilized bead carrier, the concentration of each LPS was significantly decreased, and the LPS adsorption activity of the Li5-025 immobilized bead carrier was confirmed. The efficiency of LPS removal of the Li5-025 immobilized bead carrier was higher than that of PMB.

In the adsorption test for Bp (Bordetella pertussis) using the Li5-025 immobilized bead carrier, 95% or more of 500 ng/mL LPS was adsorbed. The percentages for Vc (Vibrio cholerae serotype Inaba 569B), Kp (Klebsiella pneumoniae), Pa (Pseudomonas aeruginosa Serotype 10), and Ec(Escherichia coli O111:B4) were 90% or more, 50% or more (approximately 80% at a maximum), 95% or more, and 95% or more, respectively. This result indicates that the Li5-025 immobilized bead carrier exhibits a high adsorption activity to LPS prepared from various bacteria.

In the adsorption test of PMB, the percentages for Bp and Vc were 95% or more and 60% or more of 500 ng/mL LPS, respectively, but little adsorption was observed for Kp and Pa. This result indicates that the adsorption activity of PMB largely depends on the types of LPS.

**[Table 10]**

| | LPS removal (%) | | |
|---|---|---|---|
| Origin of LPS | 5 min | 30 min | 60 min |
| Bp | 98.4 | 96.4 | 97.1 |
| Vc | 68.1 | 89.0 | 92.1 |
| Kp | 3.3 | 42.0 | 50.1 |
| Ec | 6.2 | 83.8 | 95.1 |

### Example 12: Evaluation of Li5-025 immobilized bead carrier in LPS adsorption at low concentration (by batch method)

The peptide Li5-025 (or PMB) immobilized bead carrier, prepared in accordance with the procedures described in Example 11, was mixed with a low concentration (1 EU/mL) of LPS solution (1 mL), to confirm the LPS adsorption activity of the peptide at a low concentration of LPS by a batch method. LPS derived from Escherichia coli O113 : H10 was used. The batch method was carried out in accordance with the procedures described in Example 10.

The result is shown in Table 11. The LPS concentration in the control was not lowered even after 60 minutes. The Li5-025 or PMB immobilized bead carrier adsorbed 70% or more of LPS (1 EU/mL) after 60 minutes, and the LPS concentration was decreased to 0.3 EU/mL or less. It was thought that more LPS could be removed by the open column method.

**[Table 11]**

| Subject to be immobilized | LPS removal (%) | | |
|---|---|---|---|
| | 5 min | 30 min | 60 min |
| Li5-025 | 55.6 | 60.0 | 72.7 |
| PMB | 33.3 | 60.0 | 72.7 |

### Example 13: Evaluation of Li5-025 immobilized bead carrier- in LPS adsorption in 1% BSA solution (by batch method)

After the peptide Li5-025 (or PMB) immobilized bead carrier, prepared in accordance with the procedures described in Example 11, was mixed with 1 mL of 1% BSA solution (LPS concentration = 500 ng/mL), the batch method was carried out to examine the LPS adsorption activity in the 1% BSA solution. LPS derived from Escherichia coli O111:B4 was used. The batch method was basically carried out in accordance with the procedures described in Example 10.

The result is shown in Table 12. The Li5-025 immobilized bead carrier adsorbed 60% or more of LPS (500 ng/mL) in the presence of 1% BSA. From the results of previously performed experiments (for example, Example 3-3) using various concentrations of peptide to be immobilized, there is a possibility of increasing the LPS adsorption activity by increasing an amount of the peptide to be immobilized.

**[Table 12]**

| Subject to be immobilized | LPS removal (%) | | |
|---|---|---|---|
| | 5 min | 30 min | 60 min |
| Li5-025 | 36.8 | 59.1 | 62.4 |
| PMB | 22.1 | 7.5 | |

### Example 14: Comparison of polylysine-like peptide with Li5-052 in LPS adsorption (by batch method)

To compare polylysine known as an LPS binding compound with the peptide Li5-025 (or PMB), the following peptide was synthesized as a polylysine-like peptide in Sigma:
Peptide K7C: KKKKKKKC (SEQ ID NO: 124)
A peptide (or PMB) immobilized bead carrier prepared in accordance with the method described in Example 11 was mixed with 2 mL of a 10 ng/mL LPS solution to determine the LPS adsorption activity by the batch method, for comparison. As the LPS, LPS derived from Escherichia coli O111:B4 was used. The procedures were basically carried out in accordance with those described in Example 11.

The result is shown in Table 13. The LPS adsorption efficiencies of the peptide Li5-025, PMB, and the peptide K7C were approximately 90%, approximately 65%, and approximately 20%, respectively. It was found that polylysine exhibited a low activity in LPS adsorption.

**[Table 13]**

| Subject to be immobilized | LPS removal (%) | | |
|---|---|---|---|
| | 5 min | 30 min | 60 min |
| Li5-025 | 81.2 | 86.0 | 88.3 |
| K7C | 18.8 | 21.9 | 20.4 |
| PMB | 28.2 | 57.9 | 67.0 |

### Example 15: Evaluation of Li5-025 and derivative thereof in LPS adsorption (1) (by batch method)

In this example, the peptide Li5-025 and the derivatives thereof shown in Table 14 were used to compare the LPS adsorption activities thereof by the batch method.

The procedures were basically carried out in accordance with those described in Example 11. As the LPS solution, a solution at an LPS concentration of 500 ng/mL (0.8 mL) was used. As the LPS, LPS derived from Escherichia coli O111:B4 was used.

**[Table 14]**

| [Table 14] | |
|---|---|
| Peptide | Amino acid sequence |
| Li5-025 | kYSSSISSIRAc |
| L15-029 | kYTTTITTIRAc |
| Li5-030 | kYTTTLTTLRAc |
| Li5-031 | kYSSSLSSLRAc |
| Li5-032 | kYSSISSSIRAc |

[In the amino acid sequences, the amino acids written in lowercase letters (k and c) are D-form amino acids.]

A part of the result is shown in Table 15. The LPS adsorption efficiencies of the peptides Li5-025, Li5-032, Li5-029, and Li5-030 were approximately 95%, approximately 85%, approximately 75%, and approximately 75% of LPS at a concentration of 500 ng/mL, respectively. It was found that the peptide Li5-025 exhibited the highest activity in LPS adsorption.

**[Table 15]**

| Subject to be immobilized | LPS removal (%) | | |
|---|---|---|---|
| | 5 min | 30 min | 60 min |
| Li5-025 | 58.7 | 93.0 | 95.2 |
| Li5-029 | 85.7 | 59.3 | 72.3 |
| Li5-030 | 0.0 | 59.3 | 74.0 |
| Li5-032 | 80.1 | 76.9 | 84.8 |

### Example 16: Evaluation of Li5-025 and derivative thereof in LPS adsorption (2) (by batch method)

In this example, the peptide Li5-025 and the derivatives thereof shown in Table 16 were used to compare the LPS adsorption activities thereof by the batch method.

The procedures were basically carried out in accordance with those described in Example 11. As the LPS solution, a solution at an LPS concentration of 10 ng/mL (2.0 mL) was used. As the LPS, LPS derived from Escherichia coli O111:B4 was used.

**[Table 16]**

| Peptide | Amino acid sequence |
|---|---|
| Li5-025 | kYSSSISSIRAc |
| Li5-I6F | kYSSSFSSIRAc |
| Li5-I9F | kYSSSISSFRAc |
| Li5-I6F I9F | kYSSSFSSFRAc |

[In the amino acid sequences, the amino acids written in lowercase letters (k and c) are D-form amino acids.]

The result is shown in Table 17. The LPS adsorption efficiency of the peptide Li5-I6F I9F was approximately 75% of LPS at a concentration of 10 ng/mL. The LPS adsorption efficiencies of the peptides Li5-025, Li5-I6F, and Li5-I9F were approximately 100% of LPS at a concentration of 10 ng/mL, and it was found that the peptides Li5-025, Li5-I6F, and Li5-I9F exhibited a extremely high activity in LPS adsorption.

**[Table 17]**

| Subject to be immobilized | LPS removal (%) | | |
|---|---|---|---|
| | 5 min | 30 min | 60 min |
| Li5-025 | 93.1 | 99. 6 | 100.0 |
| Li5-I6F | 98.4 | 100.0 | 100.0 |
| Li5-I9F | 93.1 | 100.0 | 100.0 |
| Li5-I6F I9F | 3.8 | 45.6 | 74.8 |

### Example 17: Evaluation of Li5-025 immobilized column in efficiency of LPS removal (1) (by open column method)

An Li5-025 immobilized column was prepared to confirm the efficiency of LPS removal by an open column method. For comparison, a PMB immobilized column and a non-immobilized column were prepared to determine the efficiency of LPS removal.

More particularly, the peptide (or PMB) immobilized column was prepared in accordance with the procedures described in Example 11. From the top of each column, 7 mL of a 10 ng/mL LPS solution was applied, and LPS eluted from the bottom of the column was sequentially collected with a volume of 0.4 mL. Amounts of LPS contained in collected fractions were determined to evaluate the efficiency of LPS removal. Column conditions were as follows:
Column: Econo-Column (BIO-RAD)
Catalog No. 737-0516, Internal diameter = 0.5 cm, Length = 15 cm, Area of bottom = 0.2 cm²
Column beads: Si-Acid Chloride beads (0.8 g)
(Column volume = 1.2 mL)
Ligand: Peptide Li5-025 or PMB (8 mg)
Endotoxin: LPS (E.coli O111:B4)
Endotoxin solution: 10 ng/mL (7 mL)
Retention volume: 0.4 mL
Flow rate: 0.5 mL/min

As a result, while the control column was saturated immediately, the Li5-025 immobilized column could remove LPS (10 ng/mL) to a concentration of 0.1 ng/mL or less. This removal efficiency remained less than 0.1 ng/mL while flowing the LPS solution. In this connection, the LPS removal efficiency of the PMB immobilized column was approximately 50%.

### Example 18: Evaluation of Li5-025 immobilized column in efficiency of LPS removal (2) (by open column method)

In this example, an Li5-025 immobilized column was prepared to confirm the LPS removal efficiency at a low concentration of LPS by the open column method. For comparison, a PMB immobilized column and a non-immobilized column were prepared.

More particularly, a peptide (or PMB) immobilized column was prepared in accordance with the method described in Example 11. From the top of each column, 7 mL of a 2 EU/mL LPS solution was applied, and LPS eluted from the bottom of the column was sequentially collected with a volume of 0.4 mL. Amounts of LPS contained in the collected samples were determined to evaluate the LPS removal efficiency at a low concentration of LPS. Column conditions were as follows: Column: Econo-Column (BIO-RAD)
Catalog No. 737-0516, Internal diameter = 0.5 cm, Length = 15 cm, Area of bottom = 0.2 cm²
Column beads: Si-Acid Chloride beads (0.8 g)
(Column volume = 1.2 mL)
Ligand: Peptide Li5-025 or PMB (8 mg)
Endotoxin: USP RSE
(LPS derived from E.coli 0113:H10; Seikagaku Corporation)
Endotoxin solution: 2 EU/mL (7 mL)
Retention volume: 0.4 mL
Flow rate: 0.5 mL/min

As a result, while the control column was saturated immediately, the Li5-025 immobilized column could remove LPS at a low concentration of 2 EU/mL to a concentration of 0.05 EU/mL or less. The result shows that even a low concentration of LPS can be further removed using the Li5-025 immobilized column. The efficiency of the PMB immobilized column in LPS removal was approximately 60%.

### Example 19: Evaluation of Li5-025 immobilized column in efficiency of LPS removal in 1% BSA solution (3) (by open column method)

In this example, an Li5-025 immobilized column was prepared to confirm the LPS removal efficiency in 1% BSA solution by the open column method. For comparison, a PMB immobilized column and a non-immobilized column were prepared.

More particularly, a peptide (or PMB) immobilized column was prepared in accordance with the method described in Example 11. From the top of each column, 7 mL of 1% BSA solution (LPS concentration = 10 ng/mL) was applied, and LPS eluted from the bottom of the column was sequentially collected with a volume of 0.4 mL. Amounts of LPS contained in the collected samples were determined to evaluate the LPS removal efficiency in the 1% BSA solution. Column conditions were as follows:
Column: Econo-Column (BIO-RAD)
Catalog No. 737-0516, Internal diameter = 0.5 cm, Length = 15 cm, Area of bottom = 0.2 cm²
Column beads: Si-Acid Chloride beads (0.8 g)
(Column volume = 1.2 mL)
Ligand: Peptide Li5-025 or PMB (8 mg)
Endotoxin: LPS (E.coli O111:B4)
Endotoxin solution: 10 ng/mL (7 mL)
Retention volume: 0.4 mL
Flow rate: 0.5 mL/min

As a result, while the control column was saturated immediately, the Li5-025 immobilized column could remove approximately 40% of LPS from the 1% BSA solution containing 10 ng/mL LPS. The LPS removal efficiency of the PMB immobilized column was similar to that of the control, and the PMB immobilized column exhibited little activity of LPS removal. It was considered that a column filled with PMB immobilized silica beads did not show the LPS removal activity in the presence of proteins other than LPS.

### Example 20: Evaluation of Li5-025 immobilized column in efficiency of LPS removal in 1% BSA solution (4) (evaluation of dependence on concentration of peptide immobilized) (by open column method)

A column in which an amount of the peptide immobilized thereon was increased was used to perform the experiment described in Example 19, and the dependence of the peptide on the concentration of the peptide was evaluated by determining the LPS removal activity in a 1% BSA solution. The experiment was carried out under the same conditions as those described in Example 19, except that the amount of the peptide immobilized was changed from 8 mg to 24 mg.

As a result, while the control column was saturated immediately, the Li5-025 immobilized column could remove approximately 90% or more of LPS from the 1% BSA solution containing 10 ng/mL LPS. This result shows that the LPS removal efficiency will be improved by increasing the amount of the peptide immobilized. The PMB immobilized column exhibited little activity of LPS removal, as with Example 18.

### Example 21: Evaluation of fine-tuned peptides in LPS neutralizing activity

The following three peptides shown in Table 18, which were obtained by fine-tuning of the peptide Li5-001, were synthesized in SIGMA Genosys to evaluate an activity of neutralizing LPS. The LPS neutralizing activities of these peptides were evaluated by mixing each peptide with LPS and measuring an amount of LPS remaining, in accordance with the following procedures. For comparison, PMB was used as a positive control. In this connection, any buffers, distilled water, equipment, and the like used in the following procedures were endotoxin-free, unless otherwise specified.

**[Table 18]**

| Peptide | Amino acid sequence |
|---|---|
| Li5-025 | kYSSSISSIRAc |
| Li5 K1dK N2del H10R C | kYSSSISSIRAC |
| Wine Opener | kYSSSISSIRGGLLLLLL |

[In the amino acid sequences, the amino acids written in lowercase letters (k and c) are D-form amino acids. The Ls (leucine) at the C-terminus of the peptide Wine Opener were amidated.]

Each peptide (or PMB) was dissolved in distilled water at a concentration of 1 mg/mL. These solutions were sequentially diluted to 1/10 with distilled water to prepare 10-fold-diluted series (up to 1 ng/mL). LPS (USP Reference Standard Endotoxin, derived from E.coli 0113:H10 strain; Seikagaku Corporation) was added to each dilution at a final concentration of 1 EU/mL, and incubated at room temperature for 30 minutes while gently stirring. After the incubation, amounts of LPS contained in these mixtures were measured with a commercially available measuring kit (Endospecy ES-50M set; Seikagaku Corporation).

As a result, it was confirmed that all peptides exhibited a concentration-dependent neutralizing activity, and that these peptides could neutralize 70% or more of 1 EU/mL LPS at a concentration of 100 µg/mL. Particularly, the peptide Wine Opener exhibited a neutralizing activity of 87% or more at a concentration of 100 ng/mL, and the activity was approximately 100 times higher than that of PMB.

### Example 22: Sensitivity of Li5-025 in LPS detection (by BIACORE system)

In this example, the sensitivity of the peptide Li5-025 in LPS measurement was confirmed using the BIACORE system (BIACORE 2000; BIACORE), and an LPS detecting method based on a surface plasmon resonance method was examined.

More particularly, 271 RU and 843 RU of the peptides Li5-025 were immobilized on flow cells (hereinafter referred to as Fc), Fc2 and Fc3, of a sensor chip (BIACORE Sensor Chip CM5;BIACORE) by thiol coupling, respectively. As a control, cysteine was immobilized on Fc1.

Various concentrations of LPS (E.coli K12 strain) solutions [concentration = 10 ng/mL, 100 ng/mL, and 1000 ng/mL; Buffer HBS (0.01 mol/L HEPES, pH 7.4, 0.15 mol/L NaCl, and 3 mmol/L EDTA)] were applied as an analyte to the sensor chip in order of an increasing concentration to carry out the measurement.

As a result, signals detected in the Fc2 were 1.7 RU (10 ng/mL), 2 RU (100 ng/mL), and 3.3 RU (1000 ng/mL), and the sensitivity was approximately 10 ng/mL. With respect to the Fc3, 3.2 RU was detected at the concentration of 10 ng/mL, and the dependence on the amount of the peptide Li5-025 immobilized on the chip was observed.

### Example 23: Staining Gram-negative bacteria with fluorescence-labeled Li5-025

The peptide Li5-025 was conjugated to fluorescein, and the resulting conjugate was used to attempt a staining of Gram-negative bacteria.

A fluorescence-labeled peptide was prepared by conjugating fluorescein to the peptide Li5-025 via the SH group of the C-terminal cysteine of the peptide. As the bacteria, Pseudomonas aeruginosa and Lactobacillus casei were used. The fully grown bacteria in liquid media were diluted to 1/100 with pure water, and the mixture thereof were spread on a slide glass. After drying, 100% ethanol was dropped on the slide glass to perform ethanol fixing.

After the fixing, 1 µg/mL fluorescein-Li5-025 was dropped on the slide glass, and the slide glass was incubated at room temperature for 15 minutes, while protected from light. The slide glass was washed with pure water, and observed by a fluorescence microscope.

The Gram-negative bacteria, Pseudomonas aeruginosa, were strongly fluorescence-stained. This result shows that this method can be used instead of the Gram staining method.

### Example 24: Removal of endotoxin in blood using Li5-025 immobilized column

A column filled with the peptide Li5-025 immobilized beads [8 mg of the peptide or PMB was immobilized on 0.8 g of beads (Si-Acid Chloride)] was used to evaluate endotoxin removal from whole blood by the open column method. As a control, a column filled with non-immobilizing beads (i.e., beads obtained by only the blocking treatment) was used.

LPS was dissolved in human whole blood at an concentration of 2 ng/mL, and 5 mL of the LPS-containing blood was applied to the column. The eluted blood was added to an RPMI medium supplemented with fresh whole blood, and the whole was incubated at 37°C for 4 hours. Endotoxin removal was evaluated in accordance with an amount of TNFα induced by LPS. Whole blood was taken from veins of three persons, and mixed with 10 units heparin.

LPS (endotoxin) induces an expression of TNFα, and thus, the LPS adsorption activity can be indirectly evaluated in accordance with an amount of TNFα induced.

The amounts of TNFα induced are shown in Table 19. In comparison with the control, the amount of TNFα induced was decreased in blood eluted from the Li5-025 immobilized beads. This result shows that LPS was removed by the column.

**[Table 19]**

| | Ave (pg/mL) | + Error (µg/mL) | - Error (µg/mL) |
|---|---|---|---|
| Control | 181.5 | 2.3 | 1.7 |
| PMB | 155.4 | 1.8 | 3.4 |
| Li5-025 | 154.5 | 12.5 | 13.7 |

### Example 25: Effect of Li5-025 derivative on treatment and prevention of sepsis

The peptide Wine Opener (hereinafter referred to as peptide WO) synthesized in Example 21 was used as an Li5-025 derivative to evaluate effects on treatment and prevention in a mouse model for endotoxemia.

C3H/HeN mice were used, and each group consisted of eight mice. A schedule for administration is as follows:
Group A: A control group. Saline was intravenously injected to mice, and saline was intravenously injected after 30 minutes from the first injection.
Group B: Saline was intravenously injected, and the peptide WO (10 mg/kg) was intravenously injected after 30 minutes from the injection of saline.
Group C: LPS (2 mg/kg) was intravenously injected, and saline was intravenously injected after 30 minutes from the injection of LPS.
Group E: LPS (2 mg/kg) was intravenously injected, and the peptide WO (10 mg/kg) was intravenously injected after 30 minutes from the injection of LPS.
Group G: A mixture of LPS (2 mg/kg) and the peptide WO (10 mg/kg) was intravenously injected.

The effect of the peptide WO was evaluated on the basis of weight loss caused by septic shock, in comparison with the weight before administration. Weight loss after 3 days and 6 days is shown by a ratio in Table 20. In Table 20, the upper value is the average of a ratio of the current weight to that before administration, and the lower values in parentheses are the upper and lower limits of deviations from the average.

**[Table 20]**

| | Group A | Group C | Group E | Group G |
|---|---|---|---|---|
| Days 3 | 1.008 | 0.812 | 0.939 | 1.030 |
| | (+0.03 -0.04) | (+0.13 -0.079) | (+0.021 -0.026) | (+0.037 -0.02) |
| Days 6 | 0.990 | 0.866 | 0.971 | 1.028 |
| | (+0.02 -0.04) | (+0.09 -0.077) | (+0.054 -0.04) | (+0.048 -0.042) |

As shown in Table 20, in the group C in which only LPS was administered, significant weight losses were observed after 3 days and after 6 days, in comparison with the control group A. In the group E in which the peptide WO was administered after 30 minutes from the LPS administration, weight loss was observed on the day of the administration, but normal weight was significantly regained after 3 and 6 days. In the group G in which the mixture of LPS and the peptide WO was administered, a significant weight loss was not observed, and this result indicated that the peptide WO exhibited a preventive effect.

In group B in which only the peptide WO was administered, a significant weight loss was observed in comparison with the control group A (data not shown), and this result indicated that the peptide WO exhibited no short term toxicity which affected weight.

### INDUSTRIAL APPLICABILITY

The LPS and/or lipid A binding agent of the present invention may be applied to, for example, the removal of LPS and/or lipid A, or the neutralization of LPS and/or lipid A.

### FREE TEXT IN SEQUENCE LISTING

The amino acid sequences of SEQ ID NOS: 1 to 124 in the sequence listing are LPS and/or lipid A binding peptides.

The amino acid sequence of SEQ ID NO: 125 in the sequence listing is a marker sequence.

The amino acids Xaa at the first and 11th positions in the amino acid sequences of SEQ ID NOS: 1, 16, 24, and 30 in the sequence listing are independently lysine, arginine, or histidine.

The amino acids Xaa at the first position in the amino acid sequences of SEQ ID NOS: 2, 5, 20 to 23, 25 to 29, 71 to 74, and 76 to 80 in the sequence listing are independently lysine, arginine, or histidine.

The amino acids Xaa at the first and 11th positions in the amino acid sequences of SEQ ID NOS: 3 and 54 to 56 in the sequence listing are independently lysine, arginine, or histidine, and the amino acids Xaa at the 2nd to 10th positions are independently arbitrary amino acids.

The amino acids Xaa at the first and 10th positions in the amino acid sequences of SEQ ID NOS: 4, 67, 75, 81, and 82 in the sequence listing are independently lysine, arginine, or histidine.

The amino acids Xaa at the first and 10th positions in the amino acid sequences of SEQ ID NOS: 6 and 106 to 108 in the sequence listing are independently lysine, arginine, or histidine, and the amino acids Xaa at the 2nd to 9th positions are independently arbitrary amino acids.

The amino acids Xaa at the 10th position in the amino acid sequences of SEQ ID NOS: 36, 37, 43, and 44 in the sequence listing are independently lysine, arginine, or histidine.

The amino acids Xaa at the 2nd to 10th positions in the amino acid sequences of SEQ ID NOS: 45 to 53 in the sequence listing are independently arbitrary amino acids.

The amino acids Xaa at the 9th position in the amino acid sequences of SEQ ID NOS: 88, 89, 95, and 96 in the sequence listing are independently lysine, arginine, or histidine.

The amino acids Xaa at the 2nd to 9th positions in the amino acid sequences of SEQ ID NOS: 97 to 105 in the sequence listing are independently arbitrary amino acids.

## Claims

1. A method of binding lipopolysaccharide and/or lipid A, said method comprising the step of bringing lipopolysaccharide and/or lipid A into contact with a peptide selected from the group consisting of
(i) a peptide exhibiting a lipopolysaccharide and/or lipid A binding activity, and comprising the amino acid sequence of SEQ ID NO: 1, or an amino acid sequence in which one to six amino acids are deleted, substituted, and/or added in the amino acid sequence of SEQ ID NO: 1,
(ii) a peptide exhibiting a lipopolysaccharide and/or lipid A binding activity, and comprising the amino acid sequence of SEQ ID NO: 2, or an amino acid sequence in which one amino acid is deleted, substituted, and/or added in the amino acid sequence of SEQ ID NO: 2,
(iii) a peptide exhibiting a lipopolysaccharide and/or lipid A binding activity, and comprising the amino acid sequence of SEQ ID NO: 4, or an amino acid sequence in which one to six amino acids are deleted, substituted, and/or added in the amino acid sequence of SEQ ID NO: 4, and
(iv) a peptide exhibiting a lipopolysaccharide and/or lipid A binding activity, and comprising the amino acid sequence of SEQ ID NO: 5,
or a derivative of the peptide, wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a non-naturally occurring amino acid having properties similar to the original amino acid, or a combination, thereof, and wherein the derivative exhibits the lipopolysaccharide and/or lipid A binding activity provided that the method is not a method for treatment of the human or animal body by surgery or therapy or a diagnostic method practised on the human or animal body.

2. A peptide or derivative thereof of claim 1, polynucleotide encoding the peptide of claim 1, or an expression vector comprising the polynucleotide, for use as a medicament.

3. A peptide or derivative thereof of claim 1, polynucleotide encoding the peptide of claim 1, or an expression vector comprising the polynucleotide, for use in a method for treating sepsis.

4. A compound for use as a medicament, wherein the compound is the peptide or derivative thereof of claim 1, a polynucleotide encoding the peptide of claim 1, or an expression vector comprising the polynucleotide and which includes therapeutically neutralizing lipopolysaccharide and/or lipid A.

5. A method of analyzing lipopolysaccharide and/or lipid A, **characterized by** using the peptide or derivative thereof of claim 1.

6. A method of analyzing Gram-negative bacteria, **characterized by** using the peptide or derivative thereof of claim 1.

7. A peptide selected from the group consisting of
(i) a peptide exhibiting a lipopolysaccharide and/or lipid A binding activity, and comprising the amino acid sequence of SEQ ID NO: 1, or an amino acid sequence in which one to six amino acids are deleted, substituted, and/or added in the amino acid sequence of SEQ ID NO: 1,
(ii) a peptide exhibiting a lipopolysaccharide and/or lipid A binding activity, and comprising the amino acid sequence of SEQ ID NO: 2, or an amino acid sequence in which one amino acid is deleted, substituted, and/or added in the amino acid sequence of SEQ ID NO: 2,
(iii) a peptide exhibiting a lipopolysaccharide and/or lipid A binding activity, and comprising the amino acid sequence of SEQ ID NO: 4, or an amino acid sequence in which one to six amino acids are deleted, substituted, and/or added in the amino acid sequence of SEQ ID NO: 4, and
(iv) a peptide exhibiting a lipopolysaccharide and/or lipid A binding activity, and comprising the amino acid sequence of SEQ ID NO: 5,
or a derivative thereof, with the proviso that the peptide is not a peptide consisting of the amino acid sequence of SEQ ID NO: 9, wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a non-naturally occurring amino acid having properties similar to the original amino acid, or a combination, thereof, and wherein the derivative exhibits the lipopolysaccharide and/or lipid A binding activity.

8. A polynucleotide encoding the peptide of claim 7, with the proviso that the peptide is not a peptide consisting of the amino acid sequence of SEQ ID NO: 9.

9. An expression vector comprising the polynucleotide of claim 8.

10. A pharmaceutical composition comprising the peptide of claim 7, a derivative thereof, a polynucleotide encoding the peptide, or an expression vector comprising the polynucleotide, and a pharmaceutically or veterinarily acceptable carrier or diluent, with the proviso that the peptide is not a peptide consisting of the amino acid sequence of SEQ ID NO: 9 wherein the derivative is a peptide obtained by a substitution of a D-form amino acid for an L-form amino acid (such as a substitution of a D-form amino acid for the N-terminal L-form amino acid, a substitution of a D-form amino acid for the C-terminal L-form amino acid, or a substitution of a D-form amino acid for an L-form amino acid at a position other than the N-terminus and the C-terminus), an acetylation of the N-terminal amino group, an amidation of the C-terminal carboxyl group, a replacement of a naturally occurring amino acid with a non-naturally occurring amino acid having properties similar to the original amino acid, or a combination, thereof, and wherein the derivative exhibits the lipopolysaccharide and/or lipid A binding activity.

## Patentansprüche

1. Ein Verfahren zur Bindung eines Lipopolysaccharids und/oder Lipids A, wobei das Verfahren den Schritt des In-Kontakt-Bringens des Lipopolysaccharids und/oder des Lipids A mit einem Peptid umfasst, ausgewählt aus der Gruppe, bestehend aus
(i) einem Peptid, das eine Lipopolysaccharid und/oder Lipid A bindende Aktivität aufweist und die Aminosäuresequenz SEQ ID Nr. 1 oder eine Aminosäuresequenz umfasst, in der eine bis sechs Aminosäuren entfernt, substituiert und/oder in der Aminosäuresequenz SEQ ID Nr. 1 hinzugefügt wurden,
(ii) einem Peptid, das eine Lipopolysaccharid und/oder Lipid A bindende Aktivität aufweist und die Aminosäuresequenz SEQ ID Nr. 2 oder eine Aminosäuresequenz umfasst, in der eine Aminosäure entfernt, substituiert und/oder in der Aminosäuresequenz SEQ ID Nr. 2, hinzugefügt wurde,
(iii) einem Peptid, das eine Lipopolysaccharid und/oder Lipid A bindende Aktivität aufweist und die Aminosäuresequenz SEQ ID Nr. 4 oder eine Aminosäuresequenz umfasst, in der ein bis sechs Aminosäuren entfernt, substituiert und/oder in der Aminosäuresequenz SEQ ID Nr. 4 hinzugefügt wurden, und
(iv) einem Peptid, das eine Lipopolysaccharid und/oder Lipid A bindende Eigenschaft aufweist und der Aminosäuresequenz SEQ ID Nr. 5 umfasst,
oder einem Derivat des Peptids, worin das Derivat ein Peptid ist, erhalten durch eine Substitution einer D-Form der Aminosäure durch eine L-Form der Aminosäure (wie durch eine Substitution einer D-Form der Aminosäure durch die N-terminale L-Form der Aminosäure, eine Substitution einer D-Form der Aminosäure durch die C-terminale L-Form der Aminosäure oder eine Substitution durch eine D-Form der Aminosäure durch eine L-Form der Aminosäure an einer anderen Position als dem N-Terminus und dem C-Terminus), eine Acetylierung der N-terminalen Aminogruppe, eine Amidierung der C-terminalen Carboxylgruppe, einen Austausch einer natürlich vorkommenden Aminosäure durch eine nicht natürlich vorkommende Aminosäure, die ähnliche Eigenschaften wie die ursprüngliche Aminosäure aufweist, oder einer Kombination davon, und worin das Derivat eine Lipopolysaccharid und/oder Lipid A bindende Aktivität aufweist, vorausgesetzt, dass das Verfahren kein Verfahren zur Behandlung des menschlichen oder des tierischen Körpers durch Operation oder Therapie oder ein diagnostisches Verfahren ist, das am menschlichen oder tierischen Körper vorgenommen wird.

2. Ein Peptid oder ein Derivat davon nach Anspruch 1, ein Polynukleotid, das ein Peptid nach Anspruch 1 codiert, oder ein Expressionsvektor, der das Polynukleotid umfasst, zur Verwendung als Medikament.

3. Ein Peptid oder ein Derivat davon nach Anspruch 1, ein Polynukleotid, das ein Peptid nach Anspruch 1 codiert, oder ein Expressionsvektor, der das Polynukleotid umfasst, zur Verwendung in einem Verfahren zur Behandlung von Sepsis.

4. Eine Verbindung zur Verwendung als Medikament, worin die Verbindung ein Peptid oder ein Derivat davon nach Anspruch 1 ist, ein Polynukleotid, das ein Peptid nach Anspruch 1 codiert, oder ein Expressionsvektor, der das Polynukleotid umfasst, und welches ein therapeutisch neutralisierendes Lipopolysaccharid und/oder Lipid A beinhaltet.

5. Eine Verfahren zur Analyse eines Lipopolysaccharids und/oder Lipids A, **gekennzeichnet durch** die Verwendung eines Peptids nach Anspruch 1 oder eines Derivats davon.

6. Ein Verfahren zur Analyse gramnegativer Bakterien, **gekennzeichnet durch** die Verwendung eines Peptides nach Anspruch 1 oder eines Derivats davon.

7. Ein Peptid, ausgewählt aus der Gruppe, bestehend aus
(i) einem Peptid, das ein Lipopolysaccharid und/oder Lipid A bindende Aktivität aufweist und die Aminosäuresequenz SEQ ID Nr. 1 oder eine Aminosäuresequenz umfasst, in der eine bis sechs Aminosäuren entfernt, substituiert und/oder in der Aminosäuresequenz SEQ ID Nr. 1 hinzugefügt wurden,
(ii) einem Peptid, das eine Lipopolysaccharid und/oder Lipid A bindende Aktivität aufweist und eine Aminosäuresequenz SEQ ID Nr. 2 oder eine Aminosäuresequenz umfasst, in der eine Aminosäure entfernt, substituiert und/oder in der Aminosäuresequenz SEQ ID Nr. 2 hinzugefügt wurde,
(iii) einem Peptid, das eine Lipopolysaccharid und/oder Lipid A bindende Aktivität aufweist und die Aminosäuresequenz SEQ ID Nr. 4 oder eine Aminosäuresequenz umfasst, in der ein bis sechs Aminosäuren entfernt, substituiert und/oder in der Aminosäuresequenz SEQ ID Nr. 4 hinzugefügt wurden, und
(iv) einem Peptid, das ein Lipopolysaccharid und/oder Lipid A bindende Aktivität aufweist und die Aminosäuresequenz SEQ ID Nr. 5 umfasst,
oder einem Derivat davon mit der Bedingung, dass das Peptid kein Peptid ist, bestehend aus der Aminosäuresequenz SEQ ID Nr. 9, worin das Derivat ein Peptid ist, erhalten durch eine Substitution der D-Form der Aminosäure durch eine L-Form der Aminosäure (wie durch eine Substitution einer D-Form der Aminosäure durch die N-terminale L-Form der Aminosäure, eine Substitution einer D-Form der Aminosäure durch die C-terminale L-Form der Aminosäure oder eine Substitution einer D-Form der Aminosäure durch eine L-Form der Aminosäure an einer anderen Position als dem N-Terminus und dem C-Terminus), eine Acetylierung der N-terminalen Aminogruppe, eine Amidierung einer C-terminalen Carboxylgruppe, einen Austausch einer natürlich vorkommenden Aminosäure durch eine nicht natürlich vorkommende Aminosäure, die ähnliche Eigenschaften wie die ursprüngliche Aminosäure aufweist, oder einer Kombination davon, und worin das Derivat eine Lipopolysaccharid und/oder Lipid A bindende Aktivität aufweist.

8. Ein Polynukleotid, codierend das Peptid nach Anspruch 7, mit der Bedingung, dass das Peptid kein Peptid ist, bestehend aus der Aminosäuresequenz SEQ ID Nr. 9.

9. Ein Expressionsvektor, umfassend das Polynukleotid nach Anspruch 8.

10. Eine pharmazeutische Zubereitung, umfassend das Peptid nach Anspruch 7, ein Derivat davon, ein Polynukleotid, das das Peptid codiert, oder einen Expressionsvektor, der das Polynukleotid umfasst, und einem pharmazeutisch oder veterinärmedizinisch akzeptablen Träger oder Verdünnungsmittel mit der Bedingung, dass das Peptid kein Peptid ist, bestehend aus der Aminosäuresequenz SEQ ID Nr. 9, worin das Derivat ein Peptid ist, erhalten durch die Substitution einer D-Form der Aminosäure durch eine L-Form der Aminosäure (wie beispielsweise durch eine Substitution einer D-Form der Aminosäure durch die N-terminale L-Form der Aminosäure, eine Substitution einer D-Form der Aminosäure durch die C-terminale L-Form der Aminosäure oder eine Substitution einer D-Form der Aminosäure durch eine L-Form der Aminosäure an einer anderen Position als dem N-Terminus und dem C-Terminus), eine Acetylierung einer N-terminalen Aminogruppe, eine Amidierung einer C-terminalen Carboxylgruppe, einen Austausch einer natürlich vorkommenden Aminosäure durch eine nicht natürlich vorkommende Aminosäure, die ähnliche Eigenschaften wie die ursprüngliche Aminosäure aufweist, oder einer Kombination davon, und worin das Derivat eine Lipopolysaccharid und/oder Lipid A bindende Aktivität aufweist.

## Revendications

1. Procédé de liaison à un lipopolysaccharide et/ou au lipide A, ledit procédé comprenant l'étape qui consiste à mettre en contact le lipopolysaccharide et/ou le lipide A avec un peptide sélectionné dans le groupe consistant en
(i) un peptide exhibant une activité de liaison à un lipopolysaccharide et/ou au lipide A, et comprenant la séquence d'acides aminés de SEQ ID NO: 1, ou une séquence d'acides aminés dans laquelle un à six acides aminés sont supprimés, substitués, et/ou ajoutés dans la séquence d'acides aminés de SEQ ID NO: 1,
(ii) un peptide exhibant une activité de liaison à un lipopolysaccharide et/ou au lipide A, et comprenant la séquence d'acides aminés de SEQ ID NO: 2, ou une séquence d'acides aminés dans laquelle un acide aminé est supprimé, substitué, et/ou ajouté dans la séquence d'acides aminés de SEQ ID NO: 2,
(iii) un peptide exhibant une activité de liaison à un lipopolysaccharide et/ou au lipide A, et comprenant la séquence d'acides aminés de SEQ ID NO: 4, ou une séquence d'acides aminés dans laquelle un à six acides aminés sont supprimés, substitués, et/ou ajoutés dans la séquence d'acides aminés de SEQ ID NO: 4, et
(iv) un peptide exhibant une activité de liaison à un lipopolysaccharide et/ou au lipide A, et comprenant la séquence d'acides aminés de SEQ ID NO: 5,
ou un dérivé du peptide, où le dérivé est un peptide obtenu par une substitution d'un acide aminé de forme D pour un acide aminé de forme L (comme une substitution d'un acide aminé de forme D pour l'acide aminé de forme L N-terminal, une substitution d'un acide aminé de forme D pour l'acide aminé de forme L C-terminal, ou une substitution d'un acide aminé de forme D pour un acide aminé de forme L à une position autre que l'extrémité N-terminale et l'extrémité C-terminale), une acétylation du groupe amino N-terminal, une amidation du groupe carboxyle C-terminal, un remplacement d'un acide aminé survenant à l'état naturel par un acide aminé ne survenant pas à l'état naturel ayant des propriétés similaires à l'acide aminé d'origine, ou une combinaison de ceux-ci, et où le dérivé exhibe l'activité de liaison à un lipopolysaccharide et/ou au lipide A à condition que le procédé ne soit pas un procédé de traitement du corps humain ou animal par une intervention chirurgicale ou une thérapie, ou une méthode de diagnostique pratiquée sur le corps humain ou animal.

2. Peptide ou dérivé de celui-ci selon la revendication 1, polynucléotide codant pour le peptide selon la revendication 1, ou vecteur d'expression comprenant le polynucléotide, pour son utilisation en tant que médicament.

3. Peptide ou dérivé de celui-ci selon la revendication 1, polynucléotide codant pour le peptide selon la revendication 1, ou vecteur d'expression comprenant le polynucléotide, pour son utilisation dans un procédé de traitement d'un sepsis.

4. Composé pour son utilisation en tant que médicament, où le composé est le peptide ou le dérivé de celui-ci selon la revendication 1, un polynucléotide codant pour le peptide selon la revendication 1, ou un vecteur d'expression comprenant le polynucléotide, et qui consiste à thérapeutiquement neutraliser un lipopolysaccharide et/ou le lipide A.

5. Procédé d'analyse d'un lipopolysaccharide et/ou du lipide A, **caractérisé par** l'utilisation du peptide ou du dérivé de celui-ci selon la revendication 1.

6. Procédé d'analyse de bactéries à Gram-négatif, **caractérisé par** l'utilisation du peptide ou du dérivé de celui-ci selon la revendication 1.

7. Peptide sélectionné dans le groupe consistant en
(i) un peptide exhibant une activité de liaison à un lipopolysaccharide et/ou au lipide A, et comprenant la séquence d'acides aminés de SEQ ID NO: 1, ou une séquence d'acides aminés dans laquelle un à six acides aminés sont supprimés, substitués, et/ou ajoutés dans la séquence d'acides aminés de SEQ ID NO: 1,
(ii) un peptide exhibant une activité de liaison à un lipopolysaccharide et/ou au lipide A, et comprenant la séquence d'acides aminés de SEQ ID NO: 2, ou une séquence d'acides aminés dans laquelle un acide aminé est supprimé, substitué, et/ou ajouté dans la séquence d'acides aminés de SEQ ID NO: 2,
(iii) un peptide exhibant une activité de liaison à un lipopolysaccharide et/ou au lipide A, et comprenant la séquence d'acides aminés de SEQ ID NO: 4, ou une séquence d'acides aminés dans laquelle un à six acides aminés sont supprimés, substitués, et/ou ajoutés dans la séquence d'acides aminés de SEQ ID NO: 4, et
(iv) un peptide exhibant une activité de liaison à un lipopolysaccharide et/ou au lipide A, et comprenant la séquence d'acides aminés de SEQ ID NO: 5,
ou un dérivé de celui-ci, à condition que le peptide ne soit pas un peptide consistant en la séquence d'acides aminés de SEQ ID NO: 9, où le dérivé est un peptide obtenu par une substitution d'un acide aminé de forme D pour un acide aminé de forme L (comme une substitution d'un acide aminé de forme D pour l'acide aminé de forme L N-terminal, une substitution d'un acide aminé de forme D pour l'acide aminé de forme L C-terminal, ou une substitution d'un acide aminé de forme D pour un acide aminé de forme L à une position autre que l'extrémité N-terminale et l'extrémité C-terminale), une acétylation du groupe amino N-terminal, une amidation du groupe carboxyle C-terminal, un remplacement d'un acide aminé survenant à l'état naturel par un acide aminé ne survenant pas à l'état naturel ayant des propriétés similaires à l'acide aminé d'origine, ou une combinaison de ceux-ci, et où le dérivé exhibe l'activité de liaison à un lipopolysaccharide et/ou au lipide A.

8. Polynucléotide codant pour le peptide selon la revendication 7, à condition que le peptide ne soit pas un peptide consistant en la séquence d'acides aminés de SEQ ID NO: 9.

9. Vecteur d'expression comprenant le polynucléotide selon la revendication 8.

10. Composition pharmaceutique comprenant le peptide selon la revendication 7, un dérivé de celui-ci, un polynucléotide codant pour le peptide, ou un vecteur d'expression comprenant le polynucléotide, et un véhicule ou diluant acceptable sur le plan pharmaceutique ou vétérinaire, à condition que le peptide ne soit pas un peptide consistant en la séquence d'acides aminés de SEQ ID NO: 9, où le dérivé est un peptide obtenu par une substitution d'un acide aminé de forme D pour un acide aminé de forme L (comme une substitution d'un acide aminé de forme D pour l'acide aminé de forme L N-terminal, une substitution d'un acide aminé de forme D pour l'acide aminé de forme L C-terminal, ou une substitution d'un acide aminé de forme D pour un acide aminé de forme L à une position autre que l'extrémité N-terminale et l'extrémité C-terminale), une acétylation du groupe amino N-terminal, une amidation du groupe carboxyle C-terminal, un remplacement d'un acide aminé survenant à l'état naturel par un acide aminé ne survenant pas à l'état naturel ayant des propriétés similaires à l'acide aminé d'origine, ou une combinaison de ceux-ci, et où le dérivé exhibe l'activité de liaison à un lipopolysaccharide et/ou au lipide A.


### SEQUENCE LISTING

<110> PEPTIDE DOOR Co., Ltd.
<120> LPS and/or lipid A binding agent and novel peptide
<130> PEP-772
<150> JP 2005-339440
   <151> 2005-11-24
<150> PCT/JP2006/315613
   <151> 2008-08-07
<160> 125
<170> PatentIn version 3.1
<210> 1
   <211> 12
   <212> PRT
   <213> Artificial Sequence
<220>
   <223> Description of Artificial Sequence: LPS and/or lipid A binding
   peptide
<220>
   <221> MISG_FEATURE
   <222> (1)..(1)
   <223> Xaa stands for Lys, Arg, or His
<220>
   <221> MISC_FEATURE
   <222> (11)..(11)
   <223> Xaa stands for Lys, Arg, or His
<400> 1
   Xaa Asn Tyr Ser Ser Ser Ile Ser Ser Ile Xaa Ala
   1               5                   10
<210> 2
   <211> 6
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<400> 2
   Xaa Asn Tyr Ser Ser Ser
   1               5
<210> 3
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11).. (11)
   <223> Xaa stands for Lys, Arg, or His
<220>
   <221> MISC_FEATURE
   <222> (2)..(10)
   <223> Xaa stands for any amino acid
<400> 3
   Xaa Xaa Xaa Xaa Xaa Xaa Xaa Xaa Xaa Xaa Xaa
   1               5                   10
<210> 4
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10).. (10)
   <223> Xaa stands for Lys, Arg, or His
<400> 4
   Xaa Tyr Ser Ser Ser Ile Ser Ser Ile Xaa Ala
   1               5                   10
<210> 5
   <211> 5
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 5
   Xaa Tyr Ser Ser Ser
   1               5
<210> 6
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10).. (10)
   <223> Xaa stands for Lys, Arg, or His
<220>
   <221> MISC_FEATURE
   <222> (2).. (9)
   <223> Xaa stands for any amino acid
<400> 6
   Xaa Xaa Xaa Xaa Xaa Xaa Xaa Xaa Xaa Xaa
   1               5                   10
<210> 7
   <211> 12
   <212> PRT
   <213> Artificial Sequence
<220>
   <223> Description of Artificial Sequence: LPS and/or lipid A binding
   peptide
<400> 7
   Lys Asn Tyr Ser Ser Ser Ile Ser Ser Ile Lys Ala
   1               5                   10
<210> 8
   <211> 12
   <212> PRT
   <213> Artificial Sequence
<220>
   <223> Description of Artificial Sequence: LPS and/or lipid A binding
   peptide
<400> 8
   Lys Asn Tyr Ser Ser Ser Ile Ser Ser Ile Arg Ala
   1 5 10
<210> 9
   <211> 12
   <212> PRT
   <213> Artificial Sequence
<220>
   <223> Description of Artificial Sequence: LPS and/or lipid A binding
   peptide
<400> 9
   Lys Asn Tyr Ser Ser Ser Ile Ser Ser Ile His Ala
   1 5 10
<210> 10
   <211> 12
   <212> PRT
   <213> Artificial Sequence
<220>
   <223> Description of Artificial Sequence: LPS and/or lipid A binding
   peptide
<400> 10
   Arg Asn Tyr Ser Ser Ser Ile Ser Ser Ile Lys Ala
   1 5 10
<210> 11
   <211> 12
   <212> PRT
   <213> Artificial Sequence
<220>
   <223> Description of Artificial Sequence: LPS and/or lipid A binding
   peptide
<400> 11
   Arg Asn Tyr Ser Ser Ser Ile Ser Ser Ile Arg Ala
   1 5 10
<210> 12
   <211> 12
   <212> PRT
   <213> Artificial Sequence
<220>
   <223> Description of Artificial Sequence: LPS and/or lipid A binding
   peptide
<400> 12
   Arg Asn Tyr Ser Ser Ser Ile Ser Ser Ile His Ala
   1 5 10
<210> 13
   <211> 12
   <212> PRT
   <213> Artificial Sequence
<220>
   <223> Description of Artificial Sequence: LPS and/or lipid A binding
   peptide
<400> 13
   His Asn Tyr Ser Ser Ser Ile Ser Ser Ile Lys Ala
   1 5 10
<210> 14
   <211> 12
   <212> PRT
   <213> Artificial Sequence
<220>
   <223> Description of Artificial Sequence: LPS and/or lipid A binding
   peptide
<400> 14
   His Asn Tyr Ser Ser Ser Ile Ser Ser Ile Arg Ala
   1 5 10
<210> 15
   <211 12
   <212> PRT
   <213> Artificial Sequence
<220>
   <223> Description of Artificial Sequence: LPS and/or lipid A binding
   peptide
<400> 15
<210> 16
   <211> 13
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11).. (11)
   <223> Xaa stands for Lys, Arg, or His
<400> 16
   Xaa Asn Tyr Ser Ser Ser Ile Ser Ser Ile Xaa Ala Cys
   1 5 10
<210> 17
   <211> 6
   <212> PRT
   <213> Artificial Sequence
<220>
   <223> Description of Artificial Sequence: LPS and/or lipid A binding
   peptide
<400> 17
   Lys Asn Tyr Ser Ser Ser
   1 5
<210> 18
   <211> 6
   <212> PRT
   <213> Artificial Sequence
<220>
   <223> Description of Artificial Sequence: LPS and/or lipid A binding
   peptide
<400> 18
   Arg Asn Tyr Ser Ser Ser
   1 5
<210> 19
   <211> 6
   <212> PRT
   <213> Artificial Sequence
<220>
   <223> Description of Artificial Sequence: LPS and/or lipid A binding
   peptide
<400> 19
   His Asn Tyr Ser Ser Ser
   1 5
<210> 20
   <211> 7
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 20
   Xaa Asn Tyr Ser Ser Ser Ile
   1 5
<210> 21
   <211> 8
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 21
   Xaa Asn Tyr Ser Ser Ser Ile Ser
   1 5
<210> 22
   <211> 9
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 22
   Xaa Asn Tyr Ser Ser Ser Ile Ser Ser
   1 5
<210> 23
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 23
   Xaa Asn Tyr Ser Ser Ser Ile Ser Ser Ile
   1 5 10
<210> 24
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11)..(11)
   <223> Xaa stands for Lys, Arg, or His
<400> 24
   Xaa Asn Tyr Ser Ser Ser Ile Ser Ser Ile Xaa
   1 5 10
<210> 25
   <211> 7
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 25
   Xaa Asn Tyr Ser Ser Ser Cys
   1 5
<210> 26
   <211> 8
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 26
   Xaa Asn Tyr Ser Ser Ser Ile Cys
   1 5
<210> 27
   <211> 9
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 27
   Xaa Asn Tyr Ser Ser Ser Ile Ser Cys
   1 5
<210> 28
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<400> 28
   Xaa Asn Tyr Ser Ser Ser Ile Ser Ser Cys
   1 5 10
<210> 29
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 29
   Xaa Asn Tyr Ser Ser Ser Ile Ser Ser Ile Cys
   1 5 10
<210> 30
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11)..(11)
   <223> Xaa stands for Lys, Arg, or His
<400> 30
   Xaa Asn Tyr Ser Ser Ser Ile Ser Ser Ile Xaa Cys
   1 5 10
<210> 31
   <211> 5
   <212> PRT
   <213> Artificial Sequence
<220>
   <223> Description of Artificial Sequence: LPS and/or lipid A binding
   peptide
<400> 31
   Asn Tyr Ser Ser Ser
   1 5
<210> 32
   <211> 6
   <212> PRT
   <213> Artificial Sequence
<220>
   <223> Description of Artificial Sequence: LPS and/or lipid A binding
   peptide
<400> 32
   Asn Tyr Ser Ser Ser Ile
   1 5
<210> 33
   <211> 7
   <212> PRT
   <213> Artificial Sequence
<220>
   <223> Description of Artificial Sequence: LPS and/or lipid A binding
   peptide
<400> 33
   Asn Tyr Ser Ser Ser Ile Ser
   1 5
<210> 34
   <211> 8
   <212> PRT
   <213> Artificial Sequence
<220>
   <223> Description of Artificial Sequence: LPS and/or lipid A binding
   peptide
<400> 34
   Asn Tyr Ser Ser Ser Ile Ser Ser
   1               5
<210> 35
   <211> 9
   <212> PRT
   <213> Artificial Sequence
<220>
   <223> Description of Artificial Sequence: LPS and/or lipid A banding
   peptide,
<400> 35
   Asn Tyr Ser Ser Ser Ile Ser Ser Ile
   1               5
<210> 36
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (10)..(10)
   <223> Xaa stands for Lys, Arg, or His
<400> 36
   Asn Tyr Ser Ser Ser Ile Ser Ser Ile Xaa
   1               5                   10
<210> 37
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (10).. (10)
   <223> Xaa stands for Lys, Arg, or His
<400> 37
   Asn Tyr Ser Ser Ser Ile Ser Ser Ile Xaa Ala
   1 5 10
<210> 38
   <211> 6
   <212> PRT
   <213> Artificial Sequence
<220>
   <223> Description of Artificial Sequence: LPS and/or lipid A binding
   peptide
<400> 38
   Asn Tyr Ser Ser Ser Cys
   1 5
<210> 39
   <211> 7
   <212> PRT
   <213> Artificial Sequence
<220>
   <223> Description of Artificial Sequence: LPS and/or lipid A binding
   peptide
<400> 39
   Asn Tyr Ser Ser Ser Ile Cys
   1 5
<210> 40
   <211> 8
   <212> PRT
   <213> Artificial Sequence
<220>
   <223> Description of Artificial Sequence: LPS and/or lipid A binding
   peptide
<400> 40
   Asn Tyr Ser Ser Ser Ile Ser Cys
   1 5
<210> 41
   <211> 9
   <212> PRT
   <213> Artificial Sequence
<220>
   <223> Description of Artificial Sequence: LPS and/or lipid A binding
   peptide
<400> 41
   Asn Tyr Ser Ser Ser Ile Ser Ser Cys
   1 5
<210> 42
   <211> 10
   <212> PRT
   <213> Artificial Sequence
<220>
   <223> Description of Artificial Sequence: LPS and/or lipid A binding
   peptide
<400> 42
   Asn Tyr Ser Ser Ser Ile Ser Ser Ile Cys
   1 5 10
<210> 43
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (10).. (10)
   <223> Xaa stands for Lys, Arg, or His
<400> 43
   Asn Tyr Ser Ser Ser Ile Ser Ser Ile Xaa Cys
   1 5 10
<210> 44
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (10).. (10)
   <223> Xaa stands for Lys, Arg, or His
<400> 44
   Asn Tyr Ser Ser Ser Ile Ser Ser Ile Xaa Ala Cys
   1 5 10
<210> 45
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 45
   Lys Xaa Xaa Xaa Xaa Xaa Xaa Xaa Xaa Xaa Lys
   1 5 10
<210> 46
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 46
   Lys Xaa Xaa Xaa Xaa Xaa Xaa Xaa Xaa Xaa Arg
   1 5 10
<210> 47
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 47
   Lys Xaa Xaa Xaa Xaa Xaa Xaa Xaa Xaa Xaa His
   1 5 10
<210> 48
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 48
   Arg Xaa Xaa Xaa Xaa Xaa Xaa Xaa Xaa Xaa Lys
   1 5 10
<210> 49
   <211> 11
   <212> PRT
   <213> Artificial Sequence
<220>
   <223> Description of Artificial Sequence: LPS and/or lipid A binding
   peptide

<220>
   <221> MISC-FEATURE
   <222> (2)..(10)
   <223> Xaa stands for any amino acid
<400> 49
   Arg Xaa Xaa Xaa Xaa Xaa Xaa Xaa Xaa Xaa Arg
   1 5 10
<210> 50
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2)..(10)
   <223> Xaa stands for any amino acid
<400> 50
   Arg Xaa Xaa Xaa Xaa Xaa Xaa Xaa Xaa Xaa His
   1 5 10
<210> 51
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2)..(10)
   <223> Xaa stands for any amino acid
<400> 51
   His Xaa Xaa Xaa Xaa Xaa Xaa Xaa Xaa Xaa Lys
   1 5 10
<210> 52
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 52
   His Xaa Xaa Xaa Xaa Xaa Xaa Xaa Xaa Xaa Arg
   1 5 10
<210> 53
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (2).. (10)
   <223> Xaa stands for any amino acid
<400> 53
   His Xaa Xaa Xaa Xaa Xaa Xaa Xaa Xaa Xaa His
   1 5 10
<210> 54
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11)..(11)
   <223> Xaa stands for Lys, Arg, or His
<220>
   <221> MISC_FEATURE
   <222> (2).. (10)
   <223> Xaa stands for any amino acid
<400> 54
   Xaa Xaa Xaa Xaa Xaa Xaa Xaa Xaa Xaa Xaa Xaa Ala
   1 5 10
<210> 55
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (11)..(11)
   <223> Xaa stands for Lys, Arg, or His
<220>
   <221> MISC_FEATURE
   <222> (2)..(10)
   <223> Xaa stands for any amino acid
<400> 55
   Xaa Xaa Xaa Xaa Xaa Xaa Xaa Xaa Xaa Xaa Xaa Cys
   1 5 10
<210> 56
   <211> 13
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg. or His
<22>
   <221> MISC_FEATURE
   <222> (11)..(11)
   <223> Xaa stands for Lys, Arg, or His
<220>
   <221> MISC_FEATURE
   <222> (2)..(10)
   <223> Xaa stands for any amino acid
<400> 56
   Xaa Xaa Xaa Xaa Xaa Xaa Xaa Xaa Xaa Xaa Xaa Ala Cys
   1 5 10
<210> 57
   <211> 9
   <212> PRT
   <213> Artificial Sequence
<220>
   <223> Description of Artificial Sequence: LPS and/or lipid A binding
   peptide
<400> 57
   Asn Tyr Ser Ser Ser Ile Ser Ser Ile
   1 5
<210> 58
   <211> 11
   <212> PRT
   <213> Artificial Sequence
<220>
   <223> Description of Artificial Sequence: LPS and/or lipid A binding
   peptide
<400> 58
   Lys Tyr Ser Ser Ser Ile Ser Ser Ile Lys Ala
   1 5 10
<210> 59
   <211> 11
   <212> PRT
   <213> Artificial Sequence
<220>
   <223> Description of Artificial Sequence: LPS and/or lipid A binding
   peptide
<400> 59
   Lys Tyr Ser Ser Ser Ile Ser Ser Ile Arg Ala
   1 5 10
<210> 60
   <211> 11
   <212> PRT
   <213> Artificial Sequence
<220>
   <223> Description of Artificial Sequence: LPS and/or lipid A binding
   peptide
<400> 60
   Lys Tyr Ser Ser Ser Ile Ser Ser Ile His Ala
   1 5 10
<210> 61
   <211> 11
   <212> PRT
   <213> Artificial Sequence
<220>
   <223> Description of Artificial Sequence: LPS and/or lipid A binding
   peptide
<400> 61
   Arg Tyr Ser Ser Ser Ile Ser Ser Ile Lys Ala
   1 5 10
<210> 62
   <211> 11
   <212> PRT
   <213> Artificial Sequence
<220>
   <223> Description of Artificial Sequence: LPS and/or lipid A binding
   peptide
<400> 62
   Arg Tyr Ser Ser Ser Ile Ser Ser Ile Arg Ala
   1 5 10
<210> 63
   <211> 11
   <212> PRT
   <213> Artificial Sequence
<220>
   <223> Description of Artificial Sequence: LPS and/or lipid A binding
   peptide
<400> 63
   Arg Tyr Ser Ser Ser Ile Ser Ser Ile His Ala
   1 5 10
<210> 64
   <211> 11
   <212> PRT
   <213> Artificial Sequence
<220>
   <223> Description of Artificial Sequence: LPS and/or lipid A binding
   peptide
<400> 64
   His Tyr Ser Ser Ser Ile Ser Ser Ile Lys Ala
   1 5 10
<210> 65
   <211> 11
   <212> PRT
   <213> Artificial Sequence
<220>
   <223> Description of Artificial Sequence: LPS and/or lipid A binding
   peptide
<400> 65
   His Tyr Ser Ser Ser Ile Ser Ser Ile Arg Ala
   1 5 10
<210> 66
   <211> 11
   <212> PRT
   <213> Artificial Sequence
<220>
   <223> Description of Artificial Sequence: LPS and/or lipid A blinding
   peptide
<400> 66
   His Tyr Ser Ser Ser Ile Ser Ser Ile His Ala
   1 5 10
<210> 67
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<220>
   <221> MISC_FEATURE
   <222> (10)..(10)
   <223> Xaa stands for Lys, Arg, or His
<400> 67
   Xaa Tyr Ser Ser Ser Ile Ser Ser Ile Xaa Ala Cys
   1 5 10
<210> 68
   <211> 5
   <212> PRT
   <213> Artificial Sequence
<220>
   <223> Description of Artificial Sequence: LPS and/or lipid A binding
   peptide
<400> 68
   Lys Tyr Ser Ser Ser
   1               5
<210> 69
   <211> 5
   <212> PRT
   <213> Artificial Sequence
<220>
   <223> Description of Artificial Sequence: LPS and/or lipid A binding
   peptide
<400> 69
   Arg Tyr Ser Ser Ser
   1               5
<210> 70
   <211> 5
   <212> PRT
   <213> Artificial Sequence
<220>
   <223> Description of Artificial Sequence: LPS and/or lipid A binding
   peptide
<400> 70
   His Tyr Ser Ser Ser
   1               5
<210> 71
   <211> 6
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 71
   Xaa Tyr Ser Ser Ser Ile
   1               5
<210> 72
   <211> 7
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<400> 72
   Xaa Tyr Ser Ser Ser Ile Ser
   1               5
<210> 73
   <211> 8
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 73
   Xaa Tyr Ser Ser Ser Ile Ser Ser
   1               5
<210> 74
   <211> 9
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 74
   Xaa Tyr Ser Ser Ser Ile Ser Ser Ile
   1               5
<210> 75
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<220>
   <221> MISC_FEATURE
   <222> (10).. (10)
   <223> Xaa stands for Lys, Arg, or His
<400> 75
   Xaa Tyr Ser Ser Ser Ile Ser Ser Ile Xaa
   1 5 10
<210> 76
   <211> 6
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 76
   Xaa Tyr Ser Ser Ser Cys
   1 5
<210> 77
   <211> 7
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 77
   Xaa Tyr Ser Ser Ser Ile Cys
   1 5
<210> 78
   <211> 8
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 78
   Xaa Tyr Ser Ser Ser Ile Ser Cys
   1 5
<210> 79
   <211> 9
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<400> 79
   Xaa Tyr Ser Ser Ser Ile Ser Ser Cys
   1 5
<210> 80
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<400> 80
   Xaa Tyr Ser Ser Ser Ile Ser Ser Ile Cys
   1 5 10
<210> 81
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10)..(10)
   <223> Xaa stands for Lys, Arg, or His
<400> 81
   Xaa Tyr Ser Ser Ser Ile Ser Ser Ile Xaa Cys
   1 5 10
<210> 82
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10)..(10)
   <223> Xaa stands for Lys, Arg, or His
<400> 82
   Xaa Tyr Ser Ser Ser Ile Ser Ser Ile Xaa Ala Cys
   1 5 10
<210> 83
   <211> 4
   <212> PRT
   <213> Artificial Sequence
<220>
   <223> Description of Artificial Sequence: LPS and/or lipid A binding
   peptide
<400> 83
   Tyr Ser Ser Ser
   1
<210> 84
   <211> 5
   <212> PRT
   <213> Artificial Sequence
<220>
   <223> Description of Artificial Sequence: LPS and/or lipid A binding
   peptide
<400> 84
   Tyr Ser Ser Ser Ile
   1 5
<210> 85
   <211> 6
   <212> PRT
   <213> Artificial Sequence
<220>
   <223> Description of Artificial Sequence: LPS and/or lipid A binding
   peptide
<400> 85
   Tyr Ser Ser Ser Ile Ser
   1 5
<210> 86
   <211> 7
   <212> PRT
   <213> Artificial Sequence
<220>
   <223> Description of Artificial Sequence: LPS and/or lipid A binding
   peptide
<400> 86
   Tyr Ser Ser Ser Ile Ser Ser
   1 5
<210> 87
   <211> 8
   <212> PRT
   <213> Artificial Sequence
<220>
   <223> Description of Artificial Sequence: LPS and/or lipid A binding
   peptide
<400> 87
   Tyr Ser Ser Ser Ile Ser Ser Ile
   1               5
<210> 88
   <211> 9
   <212> PRT
   <213> Artificial Sequence
<220>
   <223> Description of Artificial Sequence: LPS and/or lipid A binding
   peptide
<220>
   <221> MISC_FEATURE
   <222> (9).. (9)
   <223> Xaa stands for Lys, Arg, or His
<400> 88
   Tyr Ser Ser Ser Ile Ser Ser Ile Xaa
   1               5
<210> 89
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (9)..(9)
   <223> Xaa stands for Lys, Arg, or His
<400> 89
   Tyr Ser Ser Ser Ile Ser Ser Ile Xaa Ala
   1               5                   10
<210> 90
   <211> 5
   <212> PRT
   <213> Artificial Sequence
<220>
   <223> Description of Artificial Sequence: LPS and/or lipid A binding
   peptide
<400> 90
   Tyr Ser Ser Ser Cys
   1               5
<210> 91
   <211> 6
   <212> PRT
   <213> Artificial Sequence
<220>
   <223> Description of Artificial Sequence: LPS and/or lipid A binding
   peptide
<400> 91
   Tyr Ser Ser Ser Ile Cys
   1               5
<210> 92
   <211> 7
   <212> PRT
   <213> Artificial Sequence
<220>
   <223> Description of Artificial Sequence: LPS and/or lipid A binding
   peptide
<400> 92
   Tyr Ser Ser Ser Ile Ser Cys
   1               5
<210> 93
   <211> 8
   <212> PRT
   <213> Artificial Sequence
<220>
   <223> Description of Artificial Sequence: LPS and/or lipid A binding
   peptide
<400> 93
   Tyr Ser Ser Ser Ile Ser Ser Cys
   1               5
<210> 94
   <211> 9
   <212> PRT
   <213> Artificial Sequence
<220>
   <223> Description of Artificial Sequence: LPS and/or lipid A binding
   peptide
<400> 94
   Tyr Ser Ser Ser Ile Ser Ser Ile Cys
   1               5
<210> 95
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (9).. (9)
   <223> Xaa stands for Lys, Arg, or His
<400> 95
   Tyr Ser Ser Ser Ile Ser Ser Ile Xaa Cys
   1               5                   10
<210> 96
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (9).. (9)
   <223> Xaa stands for Lys, Arg, or His
<400> 96
   Tyr Ser Ser Ser Ile Ser Ser Ile Xaa Ala Cys
   1               5                   10
<214> 97
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2)..(9)
   <223> Xaa stands for any amino acid
<400> 97
<210> 98
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2).. (9)
   <223> Xaa stands for any amino acid
<400> 98
   Lys Xaa Xaa Xaa Xaa Xaa Xaa Xaa Xaa Arg
   1 5 10
<210> 99
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2).. (9)
   <223> Xaa stands for any amino acid
<400> 99
   Lys Xaa Xaa Xaa Xaa Xaa Xaa Xaa Xaa His
   1 5 10
<210> 100
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2)..(9)
   <223> Xaa stands for any amino acid
<400> 100
   Arg Xaa Xaa Xaa Xaa Xaa Xaa Xaa Xaa Lys
   1 5 10
<210> 101
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2)..(9)
   <223> Xaa stands for any amino acid
<400> 101
   Arg Xaa Xaa Xaa Xaa Xaa Xaa Xaa Xaa Arg
   1 5 10
<210> 102
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2)..(9)
   <223> Xaa stands for any amino acid
<400> 102
   Arg Xaa Xaa Xaa Xaa Xaa Xaa Xaa Xaa His
   1 5 10
<210> 103
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2).. (9)
   <223> Xaa stands for any amino acid
<400> 103
   His Xaa Xaa Xaa Xaa Xaa Xaa Xaa Xaa Lys
   1 5 10
<210> 104
   <211> 10
   <212> PRT
   <213> Artificial Sequence
<220>
   <223> Description of Artificial Sequence: LPS and/or lipid A binding
   peptide
<220>
   <221> MISC_FEATURE
   <222> (2)..(9)
   <223> Xaa stands for any amino acid
<400> 104
   His Xaa Xaa Xaa Xaa Xaa Xaa Xaa Xaa Arg
   1 5 10
<210> 105
   <211> 10
   <212> PRT
   <213> Artificial Sequence
<220>
   <223> Description of Artificial Sequence: LPS and/or lipid A binding
   peptide
<220>
   <221> M)SC_FEATURE
   <222> (2)..(9)
   <223> Xaa stands for any amino acid
<400> 105
   His Xaa Xaa Xaa Xaa Xaa Xaa Xaa Xaa His
   1 5 10
<210> 106
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10)..(10)
   <223> Xaa stands for Lys, Arg, or His
<220>
   <221> MISC_FEATURE
   <222> (2)..(9)
   <223> Xaa stands for any amino acid
<400> 106
   Xaa Xaa Xaa Xaa Xaa Xaa Xaa Xaa Xaa Xaa Ala
   1               5                   10
<210> 107
   <211> 11
   <212> PRT
   <213> Artificial Sequence
<220>
   <223> Description of Artificial Sequence: LPS and/or lipid A binding
   peptide
<220>
   <221> MISC_FEATURE
   <222> (1)..(1)
   <223> Xaa stands for Lys, Arg, or His
<220>
   <221> MISC_FEATURE
   <222> (10).. (10)
   <223> Xaa stands for Lys, Arg, or His
<220>
   <221> MISC-FEATURE
   <222> (2)..(9)
   <223> Xaa stands for any amino acid
<400> 107
   Xaa Xaa Xaa Xaa Xaa Xaa Xaa Xaa Xaa Xaa Cys
   1 5 10
<210> 108
   <211> 12
   <212> PRT
   <213> Artificial Sequence
<220>
   <223> Description of Artificial Sequence: LPS and/or lipid A binding
   peptide
<220>
   <221> MISC_FEATURE
   <222> (1).. (1)
   <223> Xaa stands for Lys, Arg, or His
<220>
   <221> MISC_FEATURE
   <222> (10)..(10)
   <223> Xaa stands for Lys, Arg, or His
<220>
   <221> MISC_FEATURE
   <222> (2).. (9)
   <223> Xaa stands for any amino acid
<400> 108
   Xaa Xaa Xaa Xaa Xaa Xaa Xaa Xaa Xaa Xaa Ala Cys
   1 5 10
<210> 109
   <211> 8
   <212> PRT
   <213> Artificial Sequence
<220>
   <223> Description of Artificial Sequence: LPS and/or lipid A binding
   peptide
<400> 109
   Tyr Ser Ser Ser Ile Ser Ser Ile
   1 5
<210> 110
   <211> 13
   <212> PRT
   <213> Artificial Sequence
<220>
   <223> Description of Artificial Sequence: LPS and/or lipid A binding
   peptide
<400> 110
   Lys Asn Tyr Ser Ser Ser Ile Ser Ser Ile His Ala Cys
   1 5 10
<210> 111
   <211> 7
   <212> PRT
   <213> Artificial Sequence
<220>
   <223> Description of Artificial Sequence: LPS and/or lipid A binding
   peptide
<400> 111
   Lys Asn Tyr Ser Ser Ser Cys
   1 5
<210> 112
   <211> 8
   <212> PRT
   <213> Artificial Sequence
<220>
   <223> Description of Artificial Sequence: LPS and/or lipid A binding
   peptide
<400> 112
   Lys Asn Tyr Ser Ser Ser Ile Cys
   1 5
<210> 113
   <211> 9
   <212> PRT
   <213> Artificial Sequence
<220>
   <223> Description of Artificial Sequence: LPS and/or lipid A binding
   peptide
<400> 113
   Lys Asn Tyr Ser Ser Ser Ile Ser Cys
   1 5
<210> 114
   <211> 10
   <212> PRT
   <213> Artificial Sequence
<220>
   <223> Description of Artificial Sequence: LPS and/or lipid A binding
   peptide
<400> 114
   Lys Asn Tyr Ser Ser Ser Ile Ser Ser Cys
   1 5 10
<210> 115
   <211> 11
   <212> PRT
   <213> Artificial Sequence
<220>
   <223> Description of Artificial Sequence: LPS and/or lipid A binding
   peptide
<400> 115
   Lys Asn Tyr Ser Ser Ser Ile Ser Ser Ile Cys
   1 5 10
<210> 116
   <211> 12
   <212> PRT
   <213> Artificial Sequence
<220>
   <223> Description of Artificial Sequence: LPS and/or lipid A binding
   peptide
<400> 116
   Lys Asn Tyr Ser Ser Ser Ile Ser Ser Ile His Cys
   1 5 10
<210> 117
   <211> 12
   <212> PRT
   <213> Artificial Sequence
<220>
   <223> Description of Artificial Sequence: LPS and/or lipid A binding
   peptide
<400> 117
   Asn Tyr Ser Ser Ser Ile Ser Ser Ile His Ala Cys
   1 5 10
<210> 118
   <211> 13
   <212> PRT
   <213> Artificial Sequence
<220>
   <223> Description of Artificial Sequence: LPS and/or lipid A binding
   peptide
<400> 118
   Lys Asn Tyr Ser Ser Ser Ile Ser Ser Ile Lys Ala Cys
   1 5 10
<210> 119
   <211> 13
   <212> PRT
   <213> Artificial Sequence
<220>
   <223> Description of Artificial Sequence: LPS and/or lipid A binding
   peptide
<400> 119
   Lys Asn Tyr Ser Ser Ser Ile Ser Ser Ile Arg Ala Cys
   1 5 10
<210> 120
   <211> 13
   <212> PRT
   <213> Artificial Sequence
<220>
   <223> Description of Artificial Sequence: LPS and/or lipid A binding
   peptide
<400> 120
   Arg Asn Tyr Ser Ser Ser Ile Ser Ser Ile Arg Ala Cys
   1 5 10
<210> 121
   <211> 13
   <212> PRT
   <213> Artificial Sequence
<220>
   <223> Description of Artificial Sequence: LPS and/or lipid A binding
   peptide
<400> 121
   Arg Asn Tyr Ser Ser Ser Ile Ser Ser Ile His Ala Cys
   1 5 10
<210> 122
   <211> 13
   <212> PRT
   <213> Artificial Sequence
<220>
   <223> Description of Artificial Sequence: LPS and/or lipid A binding
   peptide
<400> 122
   His Asn Tyr Ser Ser Ser Ile Ser Ser Ile His Ala Cys
   1 5 10
<210> 123
   <211> 12
   <212> PRT
   <213> Artificial Sequence
<220>
   <223> Description of Artificial Sequence: LPS and/or lipid A binding
   peptide
<400> 123
   Lys Tyr Ser Ser Ser Ile Ser Ser Ile Arg Ala Cys
   1 5 10
<210> 124
   <211> 8
   <212> PRT
   <213> Artificial Sequence
<220>
   <223> Description of Artificial Sequence: LPS and/or lipid A binding
   peptide
<400> 124
   Lys Lys Lys Lys Lys Lys Lys Cys
   1 5
<210> 125
   <211> 9
   <212> PRT
   <213> Artificial Sequence
<220>
   <223> Description of Artificial Sequence: Marker sequence
<400> 125
   Gly Gly Leu Leu Leu Leu Leu Leu Leu
   1 5